# EUROPEAN PATENT SPECIFICATION

(11) **EP 3 962 494 B1**
(45) Date of publication and mention of the grant of the patent: **04.03.2026**
(21) Application number: 20722577.2
(22) Date of filing: 29.04.2020
(51) Int. Cl.: A61K 31/7105, A61K 38/09, A61P 25/16, A61P 25/28, A61P 43/00

(54) **PULSATIVE GNRH ADMINISTRATION FOR USE IN TREATING COGNITIVE DISORDERS**
PULSIERENDE GNRH-VERABREICHUNG ZUR VERWENDUNG BEI DER BEHANDLUNG VON KOGNITIVEN STÖRUNGEN
ADMINISTRATION DE GNRH PULSÉE POUR UTILISATION DANS LE TRAITEMENT DE TROUBLES COGNITIFS

(30) Priority: 30.04.2019 EP 19305550
(43) Date of publication of application: 09.03.2022
(73) Proprietor: INSERM (Institut National de la Santé et de la Recherche Médicale), 75013 Paris (FR); Université de Lille, 59000 Lille (FR); Centre Hospitalier Régional et Universitaire de Lille, 59000 Lille (FR)
(72) Inventor: PREVOT, Vincent, 59000 LILLE (FR); MESSINA, Andrea, 1005 Lausanne (CH); GIACOBINI, Paolo, 59000 LILLE (FR); LEYSEN, Valérie, 59000 LILLE (FR); MANFREDI LOZANO, Maria, 59000 LILLE (FR)
(74) Representative: Plasseraud IP
(86) International application number: PCT/EP2020/061943
(87) International publication number: WO 2020/221821

(56) References cited:
- WO-A1-2017/182580
- WO-A2-2007/041386
- WO-A2-2014/179139
- CN-A- 108 837 143
- US-A1- 2008 171 736
- EIBENSTEIN A ET AL: "Modern psychophysical tests to assess olfactory function", NEUROLOGICAL SCIENCES ; OFFICIAL JOURNAL OF THE ITALIAN NEUROLOGICAL SOCIETY, SPRINGER-VERLAG, MI, vol. 26, no. 3, July 2005 (2005-07-01), pages 147 - 155, XP019361574, ISSN: 1590-3478
- BALLARD CLIVE ET AL: "Dementia in Down's syndrome", LANCET NEUROLOGY, LANCET PUBLISHING GROUP, LONDON, GB, vol. 15, no. 6, 11 April 2016 (2016-04-11), pages 622 - 636, XP029500780, ISSN: 1474-4422, DOI: 10.1016/S1474-4422(16)00063-6

## Description

### Field of the invention

The present invention pertains to novel therapeutic ways for treating cognitive disorders associated with olfactory dysfunction. The present invention particularly pertains to the pulsatile administration of the gonadotropin-releasing hormone (GnRH) for the treatment of cognitive disorders associated with olfactory dysfunction.

### Background of the invention

The association of olfactory and cognitive impairments can be found in several disorders. Olfactory dysfunction has e.g. been shown during Alzheimer disease, Parkinson disease, dementia, Down syndrome, or non-Down syndrome retardation (Doty, 2012), suggesting that a common pathological substrate may be involved in these diseases. However, the mechanisms responsible for this olfactory impairment remain unknown.

Cognitive disorders generally involve memory impairment as well as an impairment of at least one other cognitive domain such as attention, language, visuospatial skills or problem solving. These impairments strongly compromise patients' daily functional activities and generally require intensive health care.

To date, there is no satisfactory standard treatment for cognitive disorders.

There is thus a need for novel therapeutic approaches for cognitive disorders.

Down syndrome (DS), also known as trisomy 21, is the most common genetic form of intellectual disability, with a prevalence of 10 to 14 per 10 000 live births worldwide, that is associated with age-dependent multi-comorbidities (Antonarakis, 2017; Bayen et al., 2018). Patients with DS indeed appear to have accelerated aging physiology and conditions that often manifest by their 40th birthday. In particular, adults with DS are at very high risk of developing Alzheimer disease (AD) partially due to overexpression of amyloid precursor protein, encoded by *APP,* as a result of the location of this gene on chromosome 21. While postmortem studies reveal that almost 100% of adults with DS show the neuropathological changes of AD by the age of 40 years (Editorial, 2013; Lott and Head, 2019), clinical studies show that, in people with DS younger than 40 years, the rate of dementia is overall low, despite lifelong intellectual impairment. However, clinical symptoms of dementia rapidly emerge from age 40 years onwards (Ballard et al., 2016).

DS is often associated with fertility and olfactory impairments. Curiously, these features can also be found in another disorder: Kallmann syndrome (KS). KS is a genetic disorder that affects both the reproductive axis and the olfactory system. It is a form of hypogonadotropic hypogonadism that is caused by a failed migration of the gonadotropin-releasing hormone (GnRH) neurons from the olfactory placode into the brain. Patients present an absence of puberty onset and fertility, and a loss of the sense of smell.

DS patients have fertility impairment and accelerated aging physiology. The natural aging process results in alteration of the hypothalamus-pituitary gonadal axis, leading first to the dramatic decline in oestrogens (Studd et al., 1978) and a gradual decline in testosterone in men (Deslypere et al., 1987). Both low oestrogens in women (Manly et al., 2000) and low testosterone in men have been consistently associated with poor cognitive performance and increased risk of AD (Moffat et al., 2004; Yaffe et al., 2002) . The dramatic decline in gonadal steroids in women is due to the loss of the ovarian reserve at menopause that triggers a marked and permanent increase in the activity of the hypothalamic neurons releasing GnRH, which is the neurohormone controlling reproduction and species survival. This increase is due to the lack of the suppressive negative feedback effect of oestrogens upon GnRH secretion and results in a dramatic increase in circulating levels of the gonadotropins released by the pituitary, including the luteinizing hormone (LH) (Studd et al., 1978; Yen and Tsai, 1971). In elderly men, the decrease in testosterone pulse frequency and serum levels is associated with an alteration of the hypothalamo-pituitary function, which may result from a gradual decrease in GnRH secretion with age (Deslypere et al., 1987). Similarly, postmenopausal elderly women show marked age-related decrease in the pulsatile activity of the hypothalamic component of the reproductive axis (Hall et al., 2000). In parallel to these studies in humans suggesting the existence of ageing-related decline in hypothalamic GnRH secretion, recent studies in mice have proposed the involvement of the GnRH neuroendocrine system in systemic aging (Zhang et al., 2013).

WO 2014/179139 concerns the use of GnRH for treating the effects of aging, including age-related cognitive decline. Mice were injected subcutaneously with GnRH at 2 ng/mouse on a daily basis for 5-8 weeks.

CN 108 837 143 discloses the use of triptorelin in the treatment and/or prevention of neurological diseases associated with cognitive decline. Triptorelin is administered once daily for 1-3 weeks.

US 2008/171736 discloses the combination of a GnRH analog in combination with an AChE inhibitor and/ or an NMDA antagonist for treating, mitigating, slowing the progression or preventing Alzheimer's disease and mild cognitive impairment. Certain GnRH analogs are administered oce or twice daily.

In view of the above, the present inventors evaluated whether acquired GnRH deficiency could play a role in the age-dependent acquisition of cognitive decline in DS (Schapiro et al., 1987).

Both GnRH neurons and olfactory neurons arise from the same group of progenitor cells in the olfactory placode and GnRH neurons migrate into the hypothalamus of the brain during further embryogenesis. This shared origin highlights a link between the GnRH system and the olfactory system. The present inventors thus further evaluated whether GnRH deficiency could be implied in the cognitive impairments found in cognitive disorders associated with olfactory dysfunction.

### Detailed description

Using a mouse model of DS (Ts65Dn), the present inventors demonstrated that the acquisition of cognitive defects, but also decline in olfactory perception, anosmia being a hallmark of both DS (Nijjar and Murphy, 2002) and dementia (Doty, 2012) as well, is accompanied by a gradual loss of GnRH expression in the brain during postnatal development and is associated with an altered pattern of pulsatile LH secretion in adulthood. The inventors further showed that inhibiting the activity of GnRH-R-expressing neurons in hippocampus and cortex induces cognitive and olfactory impairments in control wild-type mice. The inventors particularly demonstrated that a pulsatile GnRH treatment, usually administered to patients with congenital hypogonadotropic hypogonadism to manage their infertility (Boehm et al., 2015), allows reversing olfactory- and cognitive-associated impairments in DS.

The present inventors further demonstrated that pulsatile GnRH treatment can allow decreasing the expression of AD-associated proteins such as TauCter in the cortex of Ts65Dn mice, and this, prior to the emergence of AD symptoms, i.e. in early stages of AD. It is noteworthy that Ts65Dn mice are also considered as a useful model of AD, due to the fact that DS patients (human or mice) are at very high risk of developing Alzheimer disease (AD) partially because of the overexpression of the *APP* gene located on chromosome 21 (or on corresponding chromosome 16 in mice).

The present application thus shows that:
- GnRH deficiency is involved in the age-dependent acquisition of cognitive decline in DS;
- Inhibition of GnRH-R expressing neurons in extrahypothalamic structures induces both cognitive and olfactory impairments;
- Pulsatile GnRH treatment allows reversing olfactory- and cognitive-associated impairments in DS; and
- GnRH treatment allows reducing the expression of AD-associated proteins, AD being known to be associated with olfactory dysfunction (see e.g. Doty, 2012).

As mentioned above, multiple cognitive disorders other than DS and AD (such as Parkinson disease, dementia or non-Down syndrome retardation) are associated with concomitant olfactory dysfunction and cognitive decline. Cognitive disorders in which the cognitive decline is associated with an olfactory dysfunction thus share a same pathological pathway.

GnRH neurons originate from olfactory progenitor cells during embryo development, and as demonstrated herein, deficiency in GnRH expression induces olfactory and cognitive impairments.

Thus, without being bound by theory, the present application shows that GnRH deficiency is involved in the pathological pathways of cognitive disorders for which cognitive decline is associated with olfactory impairment.

The present application thus demonstrates that a GnRH substitution treatment allows reversing olfactory and cognitive impairments in cognitive disorders.

Accordingly, the present invention pertains to GnRH for use in the treatment of a cognitive disorder in a patient in need thereof, wherein said GnRH is administered by pulsatile administration.

The present invention particularly pertains to GnRH for use in the treatment of a cognitive disorder in a patient in need thereof, wherein said GnRH is administered by pulsatile administration and wherein said patient has an olfactory dysfunction.

GnRH is a neurohormone released in a pulsatile manner from GnRH neurons located in the hypothalamus. GnRH expression controls luteinizing hormone (LH) and follicle-stimulating hormone (FSH) secretion from the anterior pituitary. Differential GnRH pulse frequencies and amplitudes alter the secretion patterns of FSH and LH. GnRH is a decapeptide. In the context of the present invention, "GnRH" refers to the above GnRH decapeptide and to any water-soluble, ionizable form of GnRH, including free base and salts thereof. In a particular embodiment, "GnRH" refers to gonadorelin, and particularly to:
- GnRH hydrochloride (HCl) commercially available as FACTREL^{®}, HRF^{®}, and LUFORAN^{®}; or
- GnRH acetate/diacetate, commercially available as LUTRELEF^{®}, LUTREPULSE^{®}, KRYPTOCUR^{®}, LHRH FERRING^{®}, LUTAMIN^{®}, RELISORM L^{®}, CYSTORELIN^{®} or RELISORM^{®}.

Gonadorelin is a synthetic decapeptide that has the same amino acid sequence as endogenous GnRH synthesized in the human hypothalamus, and thus has the same pharmacological and toxicological profile as endogenous GnRH.

In the context of the present invention, the GnRH is administered in a "pulsatile" manner. As mentioned above, GnRH is naturally secreted with a specific pulse frequency and amplitude. Said frequency and amplitude vary according to species, genders and age. In the context of the present invention, the "pulsatile" administration reproduces the natural endogenous GnRH pulsatile peaks of a middle-age adult (i.e. between 20 and 30 years-old for humans) of the same species and gender as the patient, i.e. the GnRH frequency and amplitude observed in a middle-age adult of the same species and gender as the patient. Pulsatile GnRH administration is commonly used for the treatment of reproductive disorders such as amenorrhea and infertility resulting from hypogonadotropic hypogonadism - as e.g. Kallmann Syndrome (see Boehm et al. 2015; or e.g. Leyendecker et al. 1980; Schoemaker et al. 1981; Reid et al. 1981; Keogh et al. 1981, Hayes et al. 2013; or the ongoing clinical study referenced in the US National Library of medicine under the accession number NCT00383656). Thus, the skilled person knows the amount/frequency of administration to be used for reaching an endogenous GnRH pulsatile peak.

Typically, human endogenous GnRH pulsatile peaks vary from 25 to 600 ng/kg per pulse, with a peak every 60 to 180 minutes (see Hayes et al. 2013).

Typically, men GnRH pulsatile peaks correspond to an administration of 10 to 40 ng/kg of GnRH every 60 to 180 minutes, particularly of 20 to 30 ng/kg of GnRH every 90 to 150 minutes. A typical GnRH pulsatile peak in men is 25ng/kg of GnRH every 120 minutes (see Boehm et al. 2015).

Typically, women GnRH pulsatile peak correspond to an administration of 50 to 100 ng/kg of GnRH every 60 to 120 minutes, particularly of 65 to 85 ng/kg of GnRH every 80 to 110 minutes. A typical GnRH pulsatile peak in women is 75 ng/kg of GnRH every 90 minutes (i.e. 3 to 10 µg every 90 minutes - see Boehm et al. 2015 or clinical study NCT00383656).

The skilled person knows how to administer said pulsatile GnRH to the patient. GnRH is typically administered via transdermal, oral, or parenteral administration. As used herein, the term "parenteral" includes subcutaneous, intravenous, intra-arterial, intraperitoneal, intrathecal, intramuscular injection as well as infusion injections. GnRH is typically combined with pharmaceutically acceptable excipients to form a therapeutic composition suitable for transdermal or parenteral administration.

The GnRH is typically administered via transdermal delivery systems such as a pump (e.g. a portable infusion pump) that delivers GnRH boluses at specific intervals so as to reproduce the above endogenous GnRH pulsatile peaks. The LUTREPULSE^{®} system produced and commercialized by Ferring Pharmaceuticals is an example of such a pump. Other suitable pumps are e.g. disclosed in the international patent application published under reference WO2007041386 or in U.S. Patents Nos. 4,722,734; 5,013,293; 5,312,325; 5,328,454; 5,336,168; and 5,372,579.

Alternatively, GnRH can be administered via grafted GnRH-producing neurons. According to this embodiment, GnRH-producing neurons are grafted to the patient so as to replace the native GnRH neurons of the patient, thereby remedying GnRH insufficiency. As demonstrated in the Example section of the present application, cell therapy based on grafting GnRH-secreting neurons allows restoring pulsatile GnRH secretion and reverses olfactory- and cognitive- associated impairments in Ts65Dn mice.

As explained in Lund et al (2013) it is possible to develop GnRH-secreting neurons from Human Pluripotent Stem Cells (hPSCs), and in particular from Human Induced Pluripotent Stem Cells (hiPSCs), e.g. hiPSCs established from healthy donor fibroblasts. The production of such GnRH-secreting neurons does not involve the destruction of human embryos.

As explained throughout the application, the present invention aims at restoring GnRH pulsatile secretion for treating cognitive disorders, particularly associated with olfactory dysfunction. GnRH expression is regulated via the action of several miRNAs. In particular, members of the miRNA-200 family and miR-155 are known to regulate Zeb1 and Cebpb, respectively, two important repressors of GnRH promoter activators (see Messina et al (2016) as well as in the international patent application published under reference WO2017/182580). The present inventors have thus demonstrated that it is possible to restore pulsatile GnRH expression in a patient by overexpressing miRNA-200 family members (referred to as "miR-200") and/or miR-155. The inventors have particularly demonstrated that hypothalamic overexpression of miR-200 resulted in a rescue of both the capacity to differentiate odors and recognize novel objects in Ts65Dn mice. Accordingly, in a further embodiment, the present invention relates to a miR-200 and/or a miR-155 for use in the treatment of a cognitive disorder in a patient in need thereof.

In a particular embodiment, the present invention pertains to a miR-200 and/or a miR-155 for use in the treatment of a cognitive disorder in a patient in need thereof, wherein the patient has an olfactory dysfunction.

A "therapeutically effective amount" is intended for a minimal amount of active agent (i.e. the miRNA) which is necessary to impart therapeutic benefit to a patient, i.e. in the present case for restoring GnRH pulsatile secretion in said patient.

MicroRNAs (miRs) are small, noncoding RNAs that are emerging as crucial regulators of biological processes. "MicroRNA", "miRNA" or "miR" means a non-coding RNA of about 18 to about 25 nucleotides in length. These miRs could originate from multiple origins including: an individual gene encoding for a miRNA, from introns of protein coding gene, or from poly-cistronic transcript that often encode multiple, closely related microRNAs.

The miR-200 family contains miR-200a (human sequence accessible under reference MI0000737 in the miR database or under reference ENSG00000207607 in the Ensembl database), miR-200b (human sequence accessible under reference MI0000342 in the miR database or under reference ENSG00000207730 in the Ensembl database), miR-200c (human sequence accessible under reference MI0000650 in the miR database or under reference ENSG00000207713 in the Ensembl database), miR-141 (human sequence accessible under reference MI0000457 in the miR database or under reference ENSG00000207708 in the Ensembl database), and miR-429 (human sequence accessible under reference MI0001641 in the miR database or under reference ENSG00000198976 in the Ensembl database). By "miR-200", it is herein referred to any miRNA of the miR200 family listed above.

MiR-155 has the sequence shown under reference MI0000681 in the miR database and under reference ENSG00000283904 in the Ensembl database.

All these miRNAs are known to the skilled person. They can be administered by means of any procedure known for the delivery of nucleic acids to the nucleus of cells in vivo so as to restore pulsatile GnRH expression in a patient in need thereof. In particular, miR-200 family members and miR-155 can be administered by using recombinant techniques. For example, a suitable vector may be inserted into a host cell and expressed in that cell so as to express the above miRs.

As used herein, the term "vector" refers to a nucleic acid molecule capable of transporting another nucleic acid to which it has been linked. One type of suitable vector is a viral vector (e.g., replication defective retroviruses, adenoviruses, lentiviruses and adeno-associated viruses (AAV).

According to the present invention, pulsatile GnRH, the miR200 and/or the miR155 are administered for the treatment of cognitive disorders. The "cognitive disorder" can be any known cognitive disorder, and particularly a cognitive disorder involving a cognitive decline associated with an olfactory dysfunction.

"Cognitive disorders", also referred to as "neurocognitive disorders" are characterized by decline from a previously attained level of cognitive functioning (see Sachdev et al. 2014), i.e. a decline in perceptual-motor function (visual perception, visuoconstructional reasoning, perceptual-motor coordination), language (object naming, word finding, fluency, grammar and syntax, receptive language), learning and memory (free recall, cued recall, recognition memory, semantic and autobiographical long term memory, implicit learning), social cognition (recognition of emotions, theory of mind, insight), complex attention (sustained attention, divided attention, selective attention, processing speed) and executive function (planning, decision making, working memory, responding to feedback, inhibition, flexibility). For review, the fifth edition of the Diagnostic and Statistical Manual of Mental Disorders (DSM-V - a reference in the field) published by the American Psychiatric Association (APA), provides a common framework for the diagnosis of neurocognitive disorders. DSM-V particularly describes the main cognitive syndromes. It divides cognitive disorders into three categories: delirium, mild and major neurocognitive disorders, and defines criteria to delineate specific aetiological subtypes of mild and major neurocognitive disorders. The principal aetiological subtypes are Alzheimer disease; frontotemporal lobar degeneration, HIV infection, Huntington disease, Lewy body disease, Parkinson disease, Prion disease, Substance and/or medication use, traumatic brain injury and vascular disease.

As mentioned above, several cognitive disorders involve a cognitive decline associated with olfactory dysfunction. As disclosed in Doty et al (2012), such cognitive disorders are e.g. Down syndrome, Alzheimer disease, Parkinson disease, dementia or non-Down syndrome retardation.

According to a particular aspect, the cognitive disorder according to the present invention is Down syndrome. According to another aspect, the cognitive disorder is Alzheimer disease. According to a further aspect, the cognitive disorder is Parkinson disease.

Furthermore, age-associated cognitive decline, and in particular mild cognitive impairment in older age is also known to be associated with early olfactory dysfunction (see Wilson et al. 2007). Accordingly, in one further aspect, the cognitive disorder according to the present invention is age-associated cognitive decline

Olfactory dysfunction generally appears in the early or in the middle stage of the cognitive disease. Accordingly, according to another embodiment, the cognitive disorder is in early stage or middle-stage. According to a particular embodiment, the cognitive disorder is in early stage.

For instance, Alzheimer disease is known to progress on a spectrum with three stages - an early, preclinical stage with no symptoms; a middle stage of mild cognitive impairment; and a final stage marked by symptoms of dementia. The early stage comprises brain changes, including amyloid accumulation and other nerve cell changes, but without significant clinical symptoms. The middle stage comprises symptoms of memory and/or other thinking problems that are greater than normal for a person's age and education, but that do not interfere with his or her independence. The final stage of AD comprises memory loss, word-finding difficulties, and visual/spatial problems significant enough to impair a person's ability to function independently (see Sperling et al. 2011). During AD, olfactory dysfunction mainly appears during the asymptomatic preclinical stage and further during the intermediate stage corresponding to "mild cognitive impairment". Accordingly, in a specific embodiment, the cognitive disorder according to the present invention is early-stage Alzheimer disease.

Olfactory dysfunction also appears during early stages of Parkinson disease (see Ross et al. 2008). Parkinson disease progresses according to five stages known as the Hoehn and Yahr Scale. The early stages of Parkinson disease according to the present invention are stages I, II and earlier (i.e. "pre-stage Parkinson disease"). Accordingly, in another specific embodiment, the cognitive disorder according to the present invention is early-stage Parkinson disease.

As shown in Wilson et al (2007), olfactory dysfunction early appears during age-associated cognitive decline. Thus, according to one further embodiment, the cognitive disorder according to the present invention is an early-stage of age-associated cognitive decline.

In addition, as explained above, DS patients show lifelong intellectual impairment, but particularly show a strong cognitive decline around 40 years of age, and dementia after 40. Accordingly, GnRH replacement therapy can be used from adolescence, or at least in young adults 18-20, to improve patients' cognitive performances and delay the onset of dementia. Accordingly, in a further embodiment, the cognitive disorder according to the present invention is "early-stage" DS, i.e. prior the age-related cognitive decline appearing in patients of 40 and above.

The cognitive disorder according to the present invention is associated with olfactory dysfunction (so as to specifically target cognitive impairments associated with GnRH deficiency). "Olfactory dysfunction" corresponds to an alteration in the sense of smell. Said alteration may be a total loss of the sense of smell, also called "anosmia", or to a partial sense of smell which is referred to as "hyposmia", or "microsmia". Multiple olfactory tests are available to the skilled person who is used to use them for evaluating a patient's olfactory function. Olfactory tests can be divided into psychophysical tests, electrophysiological tests, and psychophysiological tests (see e.g. Doty et al (2007); Eibenstein et al (2005) and Kobal et al (1994)). An example of test implies presenting familiar odorants to the patient who then has to choose the name of the odour from a list of options. Threshold tests can also be used. They aim at determining the lowest concentration of an odorant that can be discerned by a patient.

In the context of the present invention the "patient" is a mammal (e.g. a dog, a cat, a pig). In a particular embodiment, the patient is a human.

As used herein, the terms "treating" or treatment" relates to reversing, alleviating, inhibiting the progress of, or preventing the disorder or condition to which such term applies, or reversing, alleviating, inhibiting the progress of, or preventing one or more symptoms of the disorder or condition to which such term applies.

The invention will be further illustrated by the following figures and examples. However, these examples and figures should not be interpreted in any way as limiting the scope of the present invention.

### BRIEF DESCRIPTION OF THE FIGURES

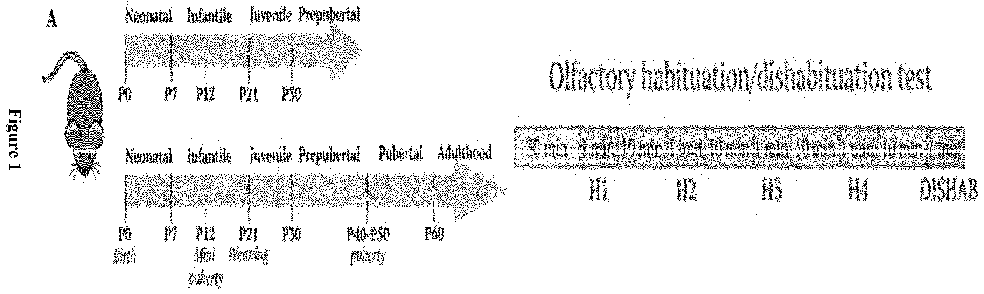

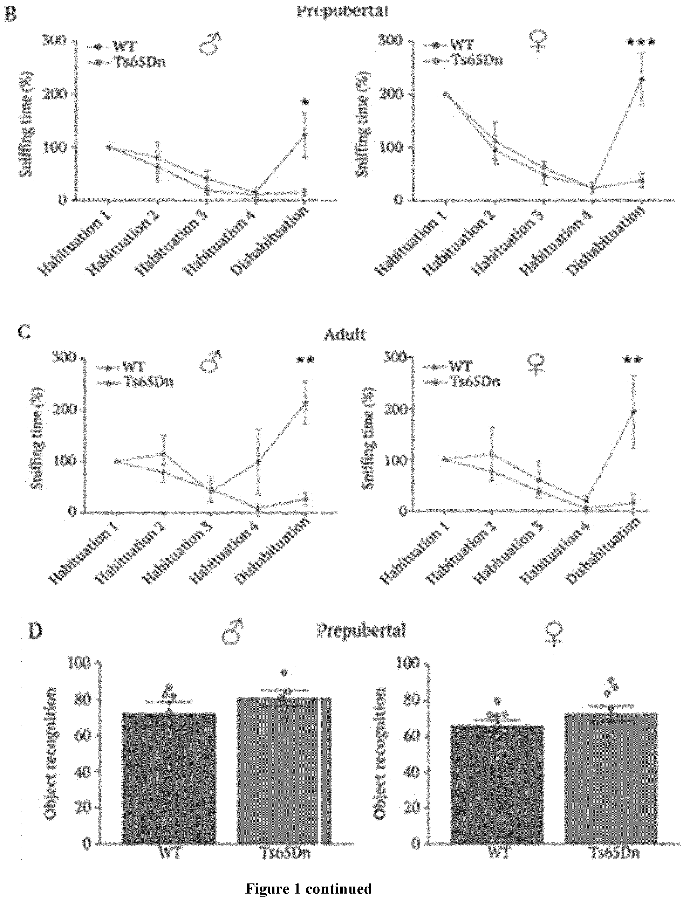

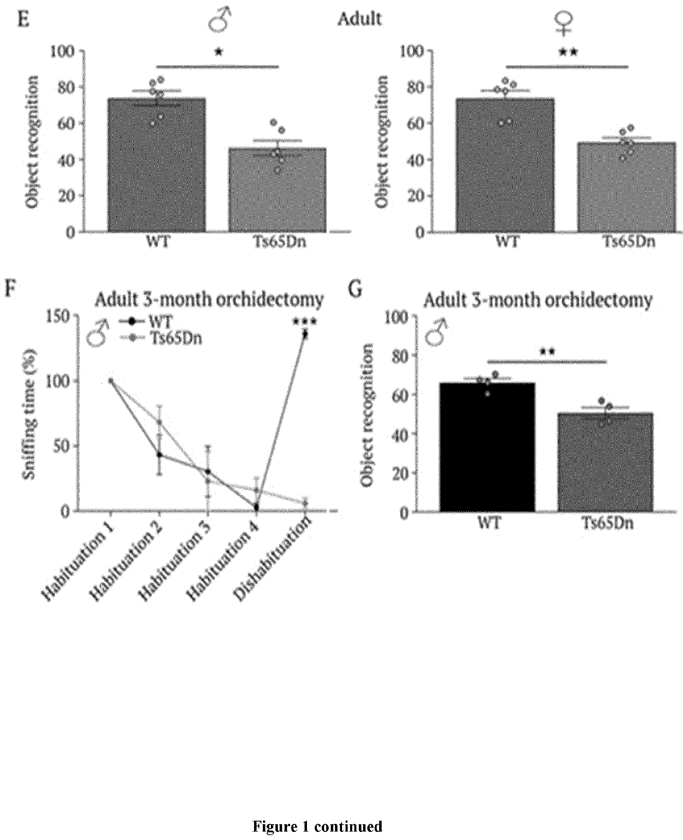

**Figure 1****: Ts65Dn mice show an age-dependent loss of the ability to recognize new odors and objects.** (A) Schematic of experimental design performed to evaluate the ability of the mice to discriminate olfactory and visual cues at different stages during postnatal development. (B) Habituation/dishabituation test was used to assess the ability to differentiate between different odors. First, one odor is presented for four consecutive times, during habituation phase; and then, a new odor is presented during the dishabituation phase. (C) Novel object recognition test was used to evaluate the recognition memory. The object recognition score was calculated as the time that the animal spent exploring a new object during the trial 2, over the total exploration time. (D) At P35, Ts65Dn mice were unable to differentiate between two distinct odors while they were equally able to recognize the introduction of new objects in their environment when compared to wild-type littermates (WT). (E) At adult ages, Ts65Dn mice showed a loss of the capacity to differentiate both different odors and objects when compared to WT littermates. * *p <* 0.05; ** *p* < 0.01.

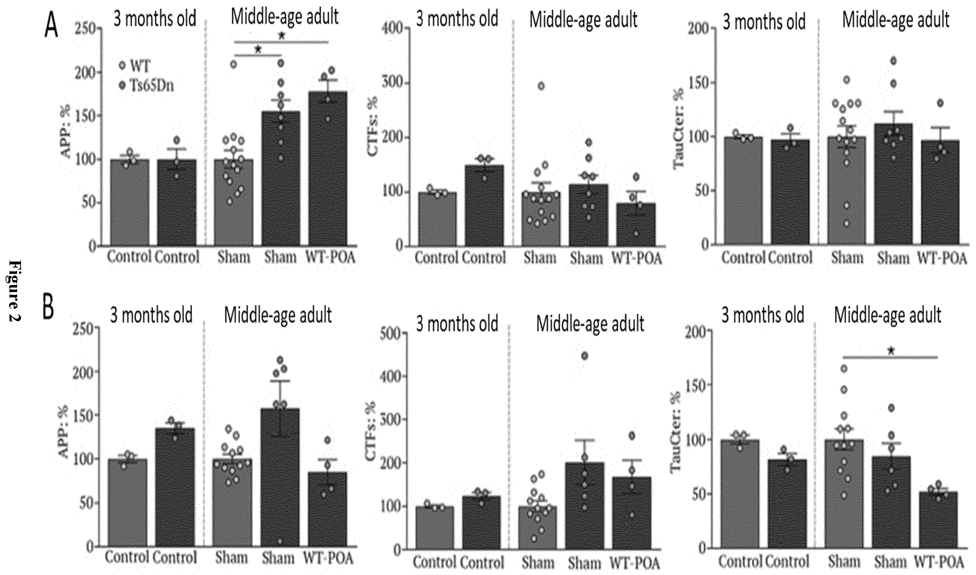

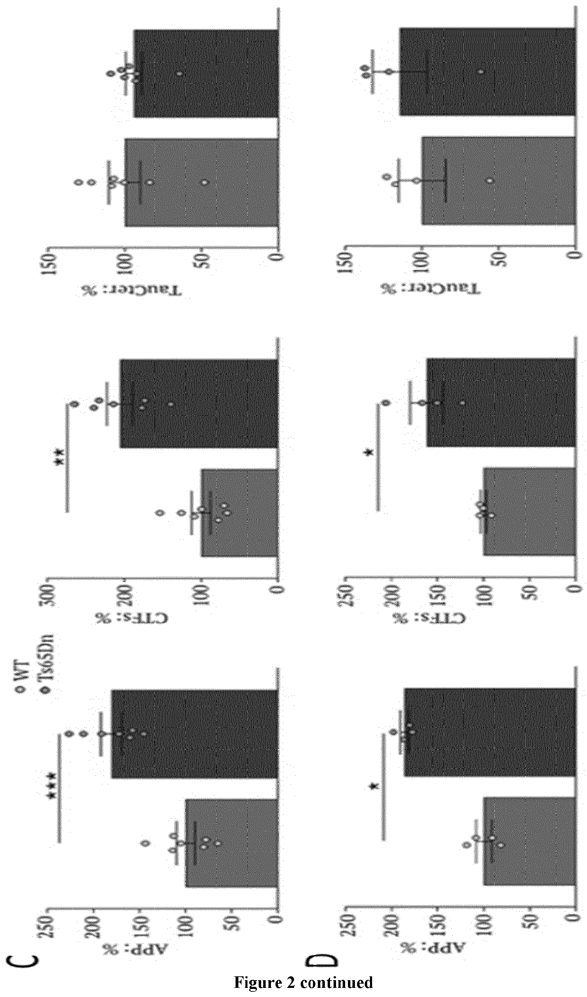

**Figure 2****: Hippocampal and cortical APP, CTF and Tau-Cter expression levels in Ts65dn mice. (A,B)** Quantification of protein levels of APP, CTF and Tau-Cter in hippocampus **(A)** and cortex **(B)** of 3-months and middle-age adult (8-12-months) Ts65dn and WT male mice, grafted with POA (WT-POA) or ungrafted (Sham). **(C,D)** Quantification of protein levels of APP, CTF and Tau-Cter in hippocampus **(C)** and cortex **(D)** of 3-months and middle-age adult (8-12-months) Ts65dn and WT female mice. GAPDH was used as a loading control. * *p <* 0.05; ** *p <* 0.01; *** *p* < 0.001.

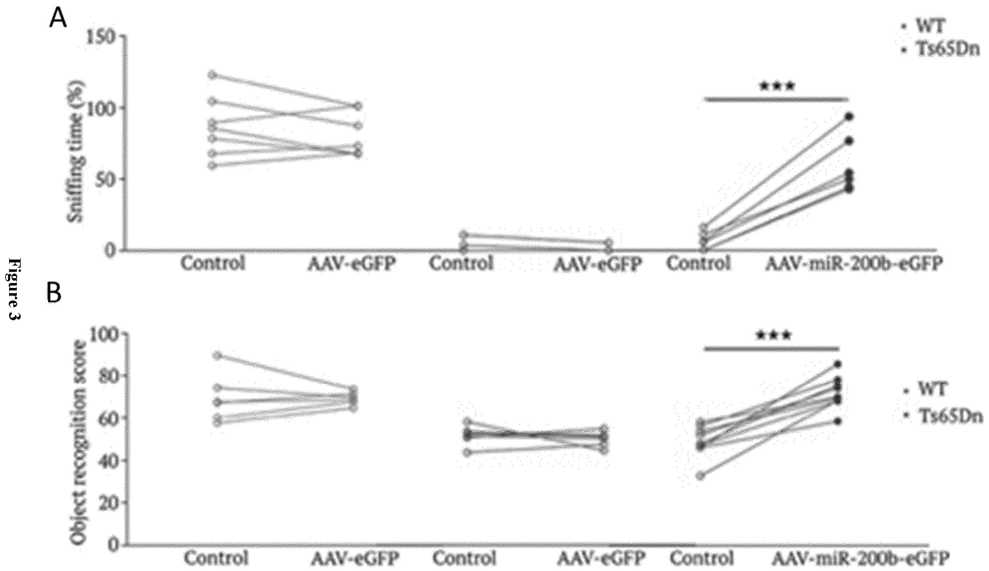

**Figure 3****: Evaluation of the functional involvement of the miR-200 family members in odor and object recognition tasks in Ts65Dn mice.** miR-200b or control miRNA were selectively overexpressed in the hypothalamus of adult male Ts65Dn mice using adeno-associated viral vectors (AAV9, Vector Biolabs). The overexpression of miR-200b resulted in a rescue of both the capacity to differentiate odors (A) and recognize novel objects (B) in Ts65Dn mice. * *p* < 0.05; ** *p* < 0.01; *** *p* < 0.001.

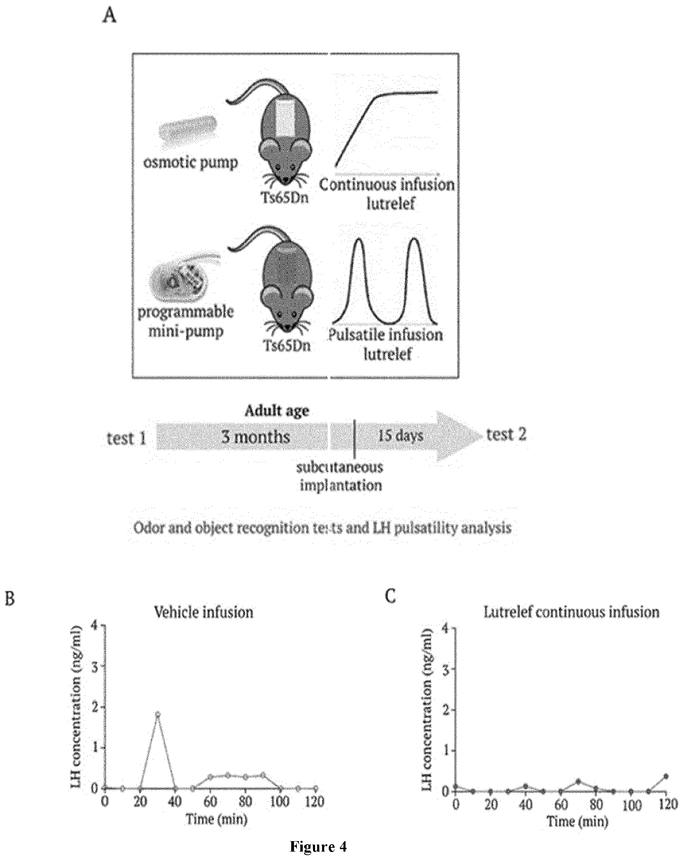

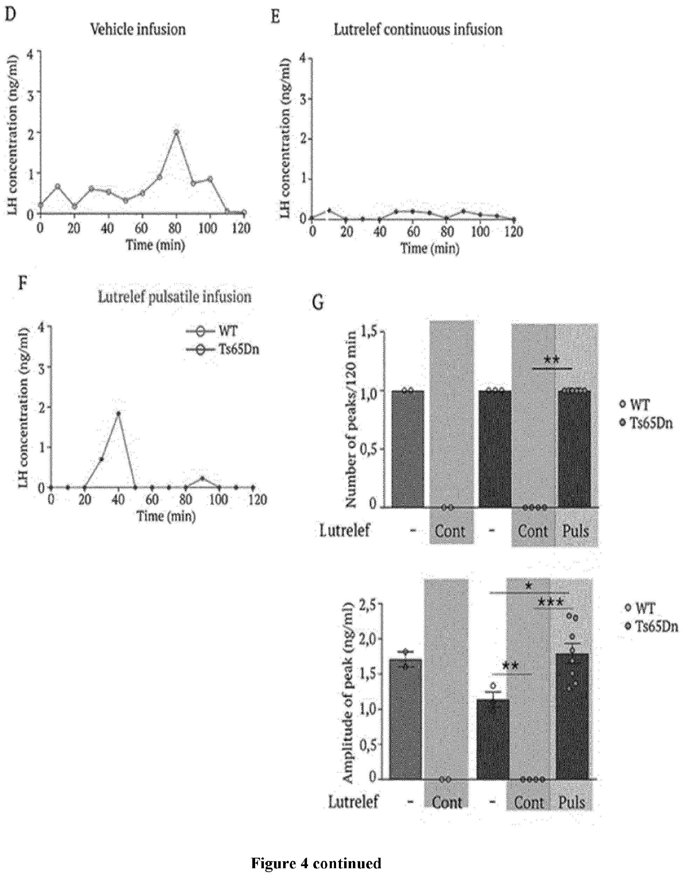

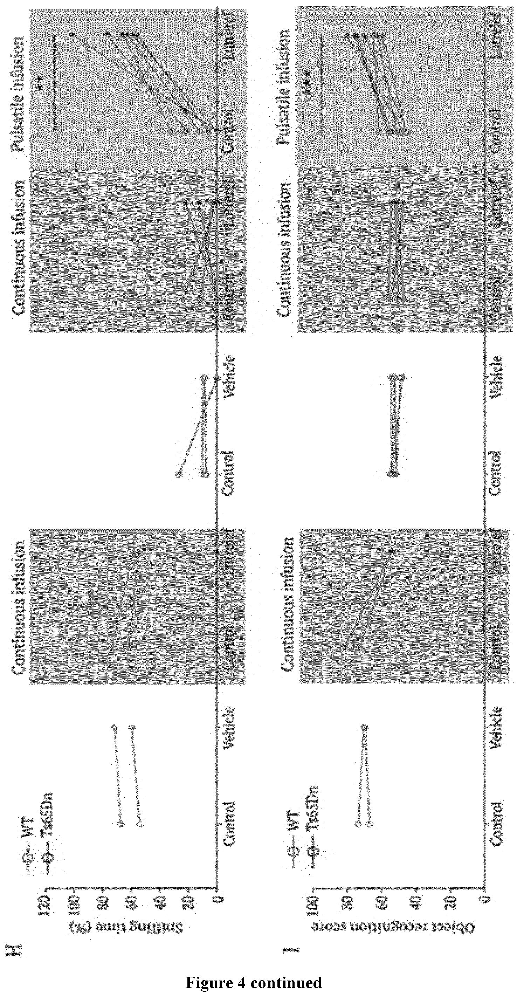

**Figure 4****: Pulsatile GnRH infusion reverses both olfactory- and cognitive-related impairments in Ts65Dn mice. (A)** Schematic diagram illustrating the pharmacological therapy performed in adult Ts65Dn mice with LUTRELEF^{®}, a GnRH peptide of clinic use. Mice were implanted with osmotic pump, to receive a continuous infusion of vehicle or LUTRELEF^{®} (0.25 µgr/ 3h); or with a programmable mini-pump (iPRECIO), to receive a pulsatile LUTRELEF^{®} infusion (every 3 hours; a peak of 0.25 µg with peak duration of 10min). **(B-F)** Representative graphs for LH pulsatility assessment after 15 days of vehicle or LUTRELEF^{®} subcutaneous administration. LUTRELEF^{®} pulsatile infusion in Ts65Dn males significantly increased LH pulse frequency and LH pulse amplitude **(G)** compared to LUTRELEF^{®} continuous infusion which prevented both LH pulse frequency and LH pulse amplitude both in WT and Ts65Dn mice **(G).** LUTRELEF^{®} pulsatile infusion rescued the capacity to discriminate between different odors **(H)** and cognitive deficits **(I)** in Ts65Dn mice. * *p <* 0.05; *** p <* 0.01; **** p <* 0.001.

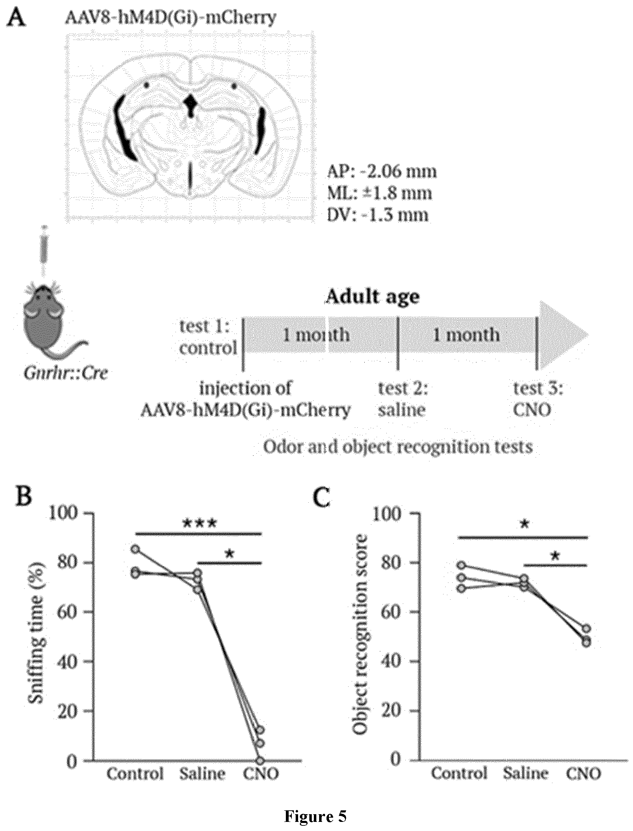

**Figure 5****: Acute chemogenetic inhibition of GnRH Receptor (GnRH-R) expressing neurons impairs cognitive and olfactory performance in adult control mice. (A)** Schematic diagram illustrating the protocol to study the effect of the chemogenetic inhibition of GnRH-R expressing neurons on cognitive and olfactory performance. Six-month old *Gnrhr::Cre* mice were tested before and after injection with an hM4D(Gi) DREADD viral vector. The two black dots indicate the injection sites of the virus. The chemogenetic inhibition of GnRH-R expressing neurons induced by clozapine-n-oxide (CNO) impaired the ability of *Gnrhr::Cre*-DREADD-injected mice to discriminate between different odors **(B)** and their cognitive performances **(C),** while saline injection had no effect **(B,C).** Every dot represents one subject. Statistical differences were tested using a one-way repeated measures ANOVA. **(B:** control vs. saline, q₍₂₎ = 1.77, *P* = 0.54, n = 3 and 3; control vs. CNO, q₍₂₎ = 60.28, *P* < 0.0001, n = 3 and 3; saline vs. CNO, q₍₂₎ = 16.92, *P* = 0.01, n = 3 and 3. **C:** control vs. saline, q₍₂₎ = 1.39, *P* = 0.65, n = 3 and 3; control vs. CNO, q₍₂₎ = 9.56, *P* = 0.04, n = 3 and 3; saline vs. CNO, q₍₂₎ = 11.27, *P* = 0.03, n = 3 and 3). * *P* < 0.05; *** *P* < 0.001.

### EXAMPLES

### Example 1

### Ts65dn male mice show delayed sexual maturation, hypogonadism and infertility.

Abnormalities of sexual development and infertility have been described in patients with Down-syndrome (DS) (Hsiang et al., 1987). Equally, an alteration of adult fertility in Ts65dn mice was previously reported, thus males show infertility and females are subfertile (Moore et al., 2010); however, sexual maturation has never been explored in this mouse model. Here, a phenotypic characterization of reproductive maturation in Ts65dn mice was performed from birth to adulthood.

Ts65dn males were smaller and presented significant lower body-weight gain than wildtype littermates during the postnatal maturation and the pubertal transition. A marked delay on puberty onset was observed in male Ts65dn mice compared with wild-type littermates. Ts65dn males exhibited a delay in the balanopreputial separation, a smaller penis and their testicles did not descend into the scrotum, all of them are external signs used to follow postnatal sexual maturation. Body weight at balanopreputial separation was identical between Ts65Dn and wild-type littermates suggesting that retarded growth may be responsible for the delay in sexual maturation. Furthermore, Ts65Dn mice presented an irregular profile of the expression of major urinary proteins expression, which excretion in the urine is stimulated by testosterone, also used as a marker of sexual maturation in mice. In adulthood, Ts65dn males revealed severe hypogonadism, exhibiting lower testicular weight and smaller testes compared with wild-type mice.

Since GnRH pulsatile secretion is essential to fertility (Belchetz et al., 1978), we next assessed the profile of secretion of the luteinizing hormone (LH), a surrogate marker of GnRH secretion, by performing serial blood sampling from the tail in adults. While no difference was found in LH pulse frequency, Ts65dn males showed significantly reduced amplitude of LH pulses when compared to their wild-type littermates. Since a deregulation on LH pulsatility can be explained by an alteration of the GnRH neuronal afferent network during postnatal development (Tata et al., 2018), glutamatergic and GABAergic appositions to GnRH neurons were analyzed. We did not find any difference in the number of appositions of vesicular glutamate transporter 2 (vGluT2)-nor GABA transporter (vGaT)-immunoreactive puncta on the soma of GnRH neurons in Ts65dn mice both at postnatal day 12 (P12) and P35, when compared with WT littermates. To further explore the function of the hypothalamus-pituitary-gonadal (HPG) axis during sexual maturation, circulating levels of gonadotropins, LH and follicle-stimulating hormone (FSH), and testosterone were measured at minipuberty (P12), i.e., the first postnatal activation of the HPG axis, and in adults. At P12, LH but not FSH levels were found to be significantly elevated in Ts65dn males when compared to wild-type littermates. In adult male DS mice, both LH and FSH levels were found to be significantly increased, whereas testosterone levels were comparable to the ones of wildtypes. These results are consistent with the findings reported in adult DS men in whom plasma testosterone levels are normal, while levels of FSH and LH are found to be significantly elevated (Hsiang et al., 1987). To probe the ability of the hypothalamus to respond to a deprivation in gonadal steroids, we next measured serum levels of testosterone and LH before (control), and 14 and 30 days after a bilateral orchiectomy. The results show that orchidectomy strongly increases LH circulating levels in both wild-type and Ts65Dn mice (data not shown). Like in intact conditions, LH levels were found to be significantly higher after orchidectomy in Ts65Dn than in wild-type littermates. Together these data indicate that the communication processes between the gonads and the hypothalamus involving gonadal steroids appear to be unaltered in Ts65Dn mice.

Phenotypic characterization of sexual maturation in the females showed that, like in males, the body-weight gain was significant lower in Ts65dn than wild-type littermates during postnatal development and pubertal transition. Ts65dn females exhibited delayed vaginal opening, an indicator of the increase in circulating estradiol levels, but no difference was found in the day of the occurrence of the first estrus, which is strictly correlated with the acquisition of reproductive capacity, i.e., puberty. Body-weight at vaginal opening and at puberty onset was lower in Ts65Dn females that in wild-type littermates. Adult Ts65dn females also presented lower uterus weight in the diestrous phase. Even though Ts65dn female mice exhibited regular estrous cyclicity, they showed attenuated fertility with fewer litters produced over a 120-day period and fewer pups per litter, when compared to wild-type littermates. However, no difference was detected in the pattern of LH secretion nor in circulating levels of FSH between Ts65dn and wild-type female mice in diestrus.

### Ts65dn mice exhibit an age-dependent loss of GnRH expression

Since a proper GnRH neuronal network development is essential for sexual maturation and correct HPG axis functioning, we next evaluated GnRH neuronal distribution in the brain of Ts65dn mice. For this purpose, we performed whole-mount immunolabeling of neonatal (P0) and adult (P90) brains for GnRH followed by three-dimensional imaging of solvent-cleared organs (3DISCO), which has previously been used to study neuronal connectivity in embryos and postnatal brains alike (Belle et al., 2017; Casoni et al., 2016). 3D analyses revealed that while there is no difference in the distribution and number of GnRH soma at birth (P0) between Ts65dn mice and WT littermates, Ts65dn mice showed a significant loss of GnRH immunoreactive soma and fibers in adulthood. The GnRH peptide content of the hypothalamus increases progressively between birth and puberty, with an acceleration of the process with the onset of minipuberty during the infantile period (P7-P12) (Messina et al., 2016; Prevot, 2015). To pinpoint the stage at which the GnRH expression starts to decrease, we next examined hypothalamic GnRH-immunoreactivity during postnatal development in Ts65dn mice. Conventional neuroanatomical analyses showed that the loss in the number of hypothalamic GnRH-immunoreactive neurons only occurred after puberty onset in Ts65Dn mice. Recent studies conducted in humans have unveiled that in addition to their distribution in the hypothalamus, GnRH soma and fibers are also found in several extrahypothalamic brain regions (Casoni et al., 2016). Accordingly, the tracing of GnRH neuronal fibers in 3D not only highlighted the classical hypophysiotropic GnRH projections in the median eminence, but also numerous GnRH neuronal projections in extra-hypothalamic regions in adult wild-type mice. GnRH-immunoreactive fibers could indeed be readily traced to the medial habenula and the anteriordorsal amygdala (Rance et al., 1994) and were often seen to be following or in close association with the wall of the lateral ventricles. However, in adult Ts65dn mice, although GnRH fibers could be visualized in the median eminence, the GnRH-immunoreactive expansive projection network seen in wildtypes was absent. The broad distribution of GnRH-immunoreactive fibers in extra-hypothalamic areas in wildtypes suggests that GnRH neurons, which control species survival, may also be involved in non-reproductive processes. Corollary, the absence of these extrahypothalamic GnRH fibers in Ts65Dn mice raise the intriguing hypothesis that this GnRH deficiency may contribute to the cognitive phenotype in this mouse model of DS.

### Ts65dn mice exhibit an age-dependent loss of olfactory and cognitive functions

DS patients and Ts65Dn mice are not only presenting mental retardation (Epstein et al., 1991; Reeves et al., 1995), but also an age-related impairment of olfaction (Bianchi et al., 2014; Nijjar and Murphy, 2002). Intriguingly, dysfunction in the ability to perceive odors is associated to GnRH deficiency in patients with Kallmann syndrome (Boehm et al., 2015). Because olfactory cues play an important role in suckling behavior (Risser and Slotnick, 1987), the sense of smell at birth in Ts65dn pups, which show normal complement of GnRH neurons and a comparable amount of milk in their stomach than wild-type littermates (not shown) did not appear to be markedly affected, in contrast to what is seen in mice harboring mutation in Kallmann genes with defective GnRH neuronal migration from nose to brain during embryogenesis and that dye at birth (Hanchate et al., 2012). To evaluate whether the loss of GnRH-immunoreactivity seen in Ts65dn mice could be associated with olfactory and cognitive decline in these mice, we performed the habituation/dishabituation test to assess the ability of the mice to discriminate between different odors (Breton-Provencher et al., 2009) and the novel object recognition test to assess recognition memory (Leger et al., 2013) in prepubertal (P35, when GnRH immunoreactivity is comparable to control littermates) and adult (>P60, when Ts65Dn mice experience a loss in GnRH immunoreactivity) mice (Fig. 1a). We found that while wild-type littermates show significantly reduced sniffing time when an odor is re-introduced (habituation), and a reinstatement of sniffing when a novel odor is presented (dishabituation); both male and female Ts65Dn mice at prepubertal age were unable to distinguish between different odors (Fig. 1b), showing a clear olfactory deficiency in these mice. In contrast, Ts65Dn mice were equally able to recognize the introduction of new objects in their environment when compared to wild-type littermates of both sexes at P35 (Fig. 1d). However, when the tests were performed two months later, i.e., in young adults, both olfactory perception and recognition memory were seen to be impaired Ts65Dn mice (Fig. 1c). These results intriguingly show the occurrence of age-related cognitive decline both in male and female Ts65Dn mice paralleling brain loss in GnRH expression (Fig. 1e). In order to determine whether cognitive decline in Ts65Dn mice could be due to aberrant gonadal function, we next evaluated olfaction and recognition memory in both wild-type and Ts65Dn mice 3 months after bilateral orchiectomy. Orchidectomized animals behaved similarly (Fig. 1f,g) than intact animals (Fig. 1c,e) suggesting that the olfactory and cognitive impairment seen in Ts65Dn mice is unlikely to be due to gonadal insufficiency or an altered communication between the brain and the gonads.

The amyloid precursor protein (App) gene triplication present in both DS patients and mice (Reeves et al., 1995) has been linked to the early-onset Alzheimer-disease (AD) phenotype observed in DS. To determine whether acquired deficiencies observed in Ts65dn mice were paralleled with the development of AD pathology in DS, we next analyzed APP, its C-terminal fragments (CTF) and Tau C-terminal (Tau-Cter) by western blot. Protein analyses revealed a significant increase in APP expression in the hippocampus (Fig. 2a) in middle-age (8-12 months), but not in young adult (3-months) Ts65dn male mice, when compared to wildtypes. In contrast, no change has been seen in the expression of CTF and Tau-Cter in the hippocampus of Ts65dn males (Fig. 2a). In the cortex, no change was seen in the expression of AD-related proteins in Ts65dn males in comparison to wild-type littermates (Fig.2 b). In females, we found a significant increase in APP and CTF expression in the hippocampus (Fig.2c) and cortex (Fig.2d) in 12-month old Ts65Dn females, while no difference is seen in Tau-Cter expression neither in the hippocampus nor in the cortex (Fig.2d). Thus, in agreement with a previous work showing age-dependent dysregulation of APP metabolism in the brain of Ts65Dn mice (Choi et al., 2009), we show that, in both sexes, the expression of APP is unaffected in young 3-month Ts65dn mice, but is seen to be increased in middle-aged animals when compared to wild-type littermates (Fig.2 a-d). Altogether these data demonstrate that the decline in olfactory perception and cognition in Ts65dn mice occurs before any obvious change in APP expression.

### Ts65dn mice show an imbalance in the miRNA-gene network controlling Gnrh expression

The loss of GnRH-immunoreactivity observed during postnatal maturation in Ts65dn mice is intriguingly reminiscent to the one seen in mice in which Dicer, an RNAse-III endonuclease essential for microRNAs biogenesis, is selectively knocked out in GnRH neurons (Messina et al., 2016). To determine whether these mice with acquired GnRH deficiency also recapitulate part of the behavioral phenotype of Ts65dn mice, we subjected adult Gnrh::Cre;DicerloxP/loxP mice to olfactory and cognitive tests. We found that the lack of mature miRNA expression in GnRH neurons leading to the postnatal loss of GnRH expression (Messina et al. 2016), also causes an impairment in the ability to discriminate odors and recognize new objects in these mice, thus phenocopying Ts65dn mice (data not shown).

It was previously reported that both the human chromosome 21 and the murine chromosome 16, the duplication of which has been used to engineer the Ts65Dn mouse line (Reeves et al. 1995), contains at least five miRNAs (miR-99a, let-7c, miR-125b-2, miR-155 and miR-802) that have been shown to be overexpressed in the DS brain (Elton et al., 2010). The effects of alteration of the copy number in these miRNAs, could thus result in the decreased expression of specific target genes and thus contribute, at least in part, to the cognitive phenotype of these individuals (Elton et al., 2010; Kuhn et al., 2010). Interestingly, we recently reported that miR-99a, let-7c, miR-125b-2 and miR-155, but not miR-802, are expressed by GnRH neurons and that their expression significantly increases between P7 and P12, i.e., at the onset of minipuberty (Messina et al., 2016). In addition, some of these miRNAs, including miR155 can also influence the expression of other miRNA species such as the expression of the members of the miR-200 family, which plays an essential role in controlling GnRH expression during postnatal development, including adulthood (Messina et al., 2016). Members of miRNA-200 family, and miR-155, are known to regulate Zeb1 and Cebpb, respectively, two important repressors of GnRH promoter activators (Messina et al., 2016). To investigate whether miRNAs are involved in the molecular mechanism underlying the postnatal loss of GnRH-immunoreactivity in Ts65dn mice, we analyzed the expression of miRNAs and different genes in the preoptic area (POA), which contains the main population of GnRH neurons in rodents, of adult wild-type and Ts65Dn littermates. Unexpectedly, real-time PCR analyses did not reveal any overexpression of miR-155, let-7c, miR-125b-2, miR-802 and miR-99a, but rather a decreased expression of these genes in the POA of Ts65dn mice (data not shown). Interestingly, data showed significant upregulation of the Zeb1 and Cebpb mRNA expression levels in the POA of adult Ts65dn mice that was accompanied by a marked decrease in Gnrh expression (Fig. 4c). This was also associated with the downregulation of the expression of most miRNA-200 family members (data not shown). In contrast, no difference was observed in the expression of Dicer, Nos1 (nitric oxide synthase 1) and its receptor, sGC (soluble guanylyl cyclase) and in the expression of several known Gnrh regulators, including Kiss1, kisspeptin receptor (Kiss1r), otx2 and meis1 (Messina et al., 2016). To gain further insights into the molecular mechanisms involved in the postnatal loss of GnRH expression in neurons of Ts65Dn mice, we constructed Gnrh::Gfp;Ts65dn reporter mice, which express GFP under an ectopic Gnrh promoter. GnRH neurons were isolated by fluorescent activated cell sorting (FACS) as was described previously (Messina et al., 2016), at P12, a developmental stage preceding the drastic drop in GnRH expression (data not shown). Real-time PCR analyses of FACS-isolated GFP-expressing GnRH neurons from Gnrh::gfp;Ts65Dn and Gnrh::gfp littermates revealed that Gnrh mRNA expression was significantly lower in Gnrh::gfp;Ts65Dn that in control littermates at P12. These altered expression levels was associated to an increase in Zeb1 expression levels and accompanied by the significant downregulation of the transcripts coding for the Gnrh promoter activator Otx2 and Kiss1r. Cebpb and Dicer expression remained unchanged. Altogether these data support the idea that the alteration of the expression of the members of the miR200 family in Ts65dn mice underlies the gradual loss of GnRH expression during postnatal development by creating an imbalance in the mir200/Zeb1/Kiss1R/Otx2 miRNA-gene micronetwork controlling Gnrh promoter activity.

To determine the putative role of the miR-200 family in the acquisition of olfactory and cognitive defects in Ts65Dn mice, miR-200b, was selectively overexpressed in the hypothalamus of adult male Ts65dn mice using stereotaxic injections of adeno-associated viral vectors (AAV). Both olfactory and cognitive performances were assessed before and after the viral infection in each one of the mice subjected to this experimental protocol. After a 3-month recovery period, the data showed that the hypothalamic overexpression of miR-200b resulted in a rescue of both the capacity to differentiate odors (Fig.3a) and recognize novel objects (Fig.3b) in Ts65Dn mice, while the Ts65Dn mice injected with the control AAV remained olfactory and memory deficient.

### GnRH replacement therapies reverse olfactory- and cognitive-associated impairments in Ts65Dn mice.

We next sought to determine whether olfactory and cognitive deficits exhibited by adult Ts65dn mice can be rescued by GnRH replacement using cell and pharmacological therapies. We first adapted a procedure previously described to restore fertility in hypogonadic mice that has demonstrated the potential of neonatal POA transplants grafting into the third ventricle (3v) to establish functional connections with host tissues (Charlton et al., 1987). Hence, neonatal cells from the POA of wild-type pups (P0-P2) were enzymatically dissociated using a papain dissociation protocol described elsewhere (Messina et al., 2016), and stereotactic injected into the 3v of adult Ts65dn mice in which both olfactory and cognitive performances were assessed before injection. After a 3-month recovery period, we observed that the implantation of wild-type neonatal preoptic tissue (WT-POA) in Ts65dn males resulted in a rescue of both olfactory and cognitive impairments when compared to Ts65Dn mice injected with vehicle solution (sham group). We also performed a Y-maze test to evaluate the short-term non-visuospatial memory in these animals. We found that in contrast to the Ts65Dn-Sham mice, the Ts65dn grafted animals spent the same amount of time in the new arm as their WT-Sham littermates. Moreover, both WT-Sham and grafted Ts65dn needed less time to first enter the new arm compared to Ts65Dn-Sham mice. Similar results were observed in adult Ts65dn females which recovered cognition after the implantation of neonatal WT-POA. However, a rescue of olfactory capacity was not observed in Ts65dn grafted females. Neonatal POA implantation did not restore the fertility in Ts65dn males (data not shown) neither estrous cyclicity in aged Ts65dn females (data not shown), showing that the restoration of olfactory capacity and cognition in these mice are uncoupled to the restoration of gonadal function. Western blot analysis revealed no change in APP and CTF expression in the cortex and the hippocampus of Ts65dn males after the implantation of neonatal POA compared with Ts65Dn control (Sham) (Fig. 2a-d), showing that the graft-mediated rescue is not associated to visible changes in these AD proteins. However, a decreased expression of the TauCter was seen in the cortex of Ts65Dn grafted with preoptic cells, when compared to sham-treated Ts65Dn mice (Fig. 2b).

In order to determine whether GnRH neurons play a role in this WT-POA-graft-mediated rescue of olfactory and cognitive functions in Ts65Dn mice, neonatal cells from Gnrh::cre; BoNTBloxP-STOP-loxP bigenic mice, a transgenic mouse line in which vesicular release in GnRH neurons is blunted by the selectively expression of the botulinum neurotoxin B in these cells (BoNTBGnrh), were injected into the 3v of adult Ts65dn males (BoNTBGnrh POA) following the protocol described above. After a 3-month recovery period, no olfactory and cognitive rescue was observed in these Ts65Dn mice grafted with BoNTBGnrh-POA cells. Interestingly, acute GnRH intraperitoneal injection (50 µg/kg of body weight) 3 month after (i.e., 6 months post-graft), was seen to rescue olfactory and cognitive deficiencies both in Sham and BoNTBGnrh POA grafted Ts65dn mice (data not shown).

GnRH neurons release their neurohormone in a pulsatile manner; pulsatile GnRH release has been monitored in vivo both in the cerebrospinal fluid (Van Vugt et al., 1985) and in the pituitary portal blood (Clarke and Cummins, 1982). Because of its presence in the CSF, a deficiency in the secretion of GnRH, in addition to impinge on the reproductive function, could also alter the function of GnRH receptor-expressing neuronal populations (Granger et al., 2004; Wilson et al., 2006) in brain areas involved in cognition both in rodents and in humans via volume transmission. As reported above, the significant change in the miRNA-gene micronetwork controlling GnRH expression, the decrease in GnRH-immunoreactivity throughout the brain, and the alteration in LH pulsatility strongly suggests that GnRH neuronal function is altered in adult Ts65Dn mice. We next explored the possibility that restoring GnRH pulsatility in Ts65dn mice is able to rescue cognitive performances in these mice. To assess this, adult Ts65dn males were implanted with osmotic pump, to receive either a continuous infusion of LUTRELEF^{®} (0.0025 µg per 10 min), a GnRH peptide of clinic use to restore fertility in patients with hypogonadotropic hypogonadism (Boehm et al., 2015); or with a programmable mini-pump, to receive a pulsatile LUTRELEF^{®} infusion (every 3 hours; a peak of 0.25 µg with peak duration of 10min) during 15 days (Fig. 4a) mimicking GnRH/LH pulsatility reported in wild-type mice (Czieselsky et al., 2016). We evaluated LH pulsatility by serial blood sampling (Fig. 4b-g); pulsatile infusion of LUTRELEF^{®} in Ts65Dn males was found to significantly increase LH pulse frequency and LH pulse amplitude (Fig. 4g) when compared with vehicle-treated Ts65Dn males. Indeed, the amplitude of LH pulses was increased to levels similar to those observed in their WT littermates (Fig. 4g). In contrast, LUTRELEF^{®} continuous infusion blunted LH pulsatility in wild-type and Ts65Dn litterates (Fig. 4g). The pulsatile infusion of LUTRELEF^{®} was seen to rescue both the capacity to discriminate between different odors (Fig. 4h) and cognitive deficits (Fig. 4i) in Ts65Dn males. While the constant infusion of LUTRELEF^{®} had no effect on olfactory and cognitive performance in Ts65Dn mice, it appeared to have marked deleterious effects on these tasks in wild-type mice (Fig. 4h,i). These data demonstrate the hitherto unsuspected importance of the pulsatile character of GnRH secretion in the beneficial effects that GnRH exerts on cognition and paves the way to the development of new treatment strategies to prevent age-dependent cognitive decline, mobilize the cognitive reserve and thus improve wellbeing in patients with neurodevelopmental (e.g., Down Syndrome) and neurodegeneradive disorders (e.g., Down Syndrome and Alzheimer disease).

### Materials and methods

### Animals

All mice were housed under specific pathogen-free conditions in a temperature controlled room (21-22°C) with a 12h light/dark cycle. The day the litters were born was considered as day 0 of age (postnatal day 0; P0). Animals were weaned at P21 and were provided with ad libitum access to food and water.

Ts65Dn (B6EiC3Sn.BLiA-Ts(1716)65Dn/DnJ; Stock no. 005252) mice (Ahmed et al., 2012; Reeves et al., 1995; Reinholdt et al., 2011) carrying a partial trisomy of chromosome 16, the orthologous region of human chromosome 21, were purchased from Jackson Laboratories (New Harbor, ME, USA). As the Ts65Dn line has a genetic background wild-type (WT) for the Pde6b gene, the line was maintained by crossing Ts65Dn trisomic females to Pde6b+ (C57BL/6JEiJ x C3Sn.BLiA-Pde6b+/DnJ)F1/J; Stock no 003647) males. This mating system results in WT and Ts65Dn animals. DicerLoxP/LoxP, Gnrh::Cre (Tg(Gnrh1::Cre)1D1c), Gnrh::Gfp and Tg(CAG-BoNT/B,EGFP)U75-56wp/J (iBot) mice were a generous gift from Dr. Brian Harfe (University of Florida, FL) (Harfe et al., 2005), Dr. Catherine Dulac (Howard Hughes Medical Institute, Cambridge MA) (Yoon et al., 2005), Dr. Daniel J. Spergel (Section of Endocrinology, Department of Medicine, University of Chicago, IL) (Spergel et al., 1999) and Dr. Frank Pfrieger (University of Strasbourg) (Slezak et al., 2012) respectively. Mice were genotyped by PCR using the primers listed in supplementary table S1. Animal studies were approved by the Institutional Ethics Committees for the Care and Use of Experimental Animals of the University of Lille; all experiments were performed in accordance with the guidelines for animal use specified by the European Union Council Directive of September 22, 2010 (2010/63/EU). The sex of the animals used is specified in the text and/or figure legends. The genotype or/and treatment group of animals was blinded for the study except when the morphological or physiological differences were too obvious to be ignored.

### Physiological measurements

**Pubertal studies.** Weaned males were checked daily for balanopreputial (BPS) separation and urine samples were collected from the weaning to P45.

Weaned female mice were checked daily for vaginal opening. After vaginal opening, vaginal smears were performed daily and analyzed under an inverted microscope to identify the specific day of estrous cycle.

**Fertility index.** Female fertility indices were calculated from the number of litters per female during a 120-day long mating.

**Hormone level measurements.** Blood collected from the submandibular vein and trunk was harvested in sterile microcentrifuge tubes and kept on ice until centrifugation. Plasma was collected after centrifugation of blood samples at 3,000 g for 15 min at 4°C and was stored at - 80°C until use.

**LH assays:** LH levels were determined by the previously described sensitive LH sandwich ELISA (Steyn et al., 2013). A 96-well high-affinity binding microplate (Corning) was coated with 50 µL of capture antibody (monoclonal antibody, anti-bovine LHβ subunit, 518B7; L. Sibley; University of California, UC Davis) at a final dilution of 1:1,000 (in 0.1M Na2CO3/NaHCO3, pH 9.6) and incubated overnight at 4°C. Wells were incubated with 200 µL blocking buffer (5% (w/v) skimmed milk powder in 1X PBS-T pH 7.4 (0.1 M PBS, 0.05% Tween 20 (Sigma #P9416)) for 2 hours at room temperature (RT). A standard curve was generated using a twofold serial dilution of mouse LH (reference preparation, AFP-5306A; National Institute of Diabetes and Digestive and Kidney Diseases National Hormone and Pituitary Program (NIDDK-NHPP)) in 1% (w/v) BSA (Sigma, A9418) in 1X PBS-T. The LH standards and blood samples were incubated with 50 µL of detection antibody (rabbit LH antiserum, AFP240580Rb; NIDDK- NHPP) at a final dilution of 1:10,000 for 1.5 hours at RT. Each well containing bound substrate was incubated with 50 µL of horseradish peroxidase-conjugated antibody (goat anti-rabbit; Vector Laboratories, PI-1000) at a final dilution of 1:10,000. After a 1.5 hours incubation, 100 µL of 1-Step Ultra TMB-Elisa Substrate Solution (ThermoFisher Scientific, cat. #34028) was added to each well and left at RT for 10 min. The reaction was stopped by the addition of 50 µL of 3M HCl to each well, and the absorbance was measured at 450 nm.

**Testosterone assays:** Plasma testosterone levels were measured using a commercial ELISA (Demeditec Diagnostics, DEV9911) (Moore et al., 2015) according to the manufacturer's instructions.

**FSH assays:** FSH levels were measured using radioimmunoassay kits supplied by the National Institutes of Health (Dr. A.F. Parlow, National Hormone and Peptide Program, Torrance, CA), as previously described in detail (Garcia-Galiano et al., 2012). Hormonal determinations were performed in duplicates. Rat FSH-I-9 was labelled with 125I by the chloramine-T method and hormone concentration was determined using reference preparations of FSH-RP-2 standards. Intra- and inter-assay coefficients of variation were less than 6 and 9% for FSH. The sensitivity of the assay was 20 pg/tube for FSH. The accuracy of hormone measurements was confirmed by the assessment of rodent serum samples of known concentration (used as external controls).

### Pulsatile LH measurements

Adult mice were habituated with daily handling. Blood samples (5 µL) were taken from the tail at 10 min intervals during a period of 2 hours (between 10:00 and 12:00) and were diluted in 45 µL of 1X PBS-T (0,05%) and immediately frozen and stored at -80°C. LH levels were then determined using the protocol described before. Pulses were confirmed using DynPeak (Vidal et al., 2012).

### Urine collection and protein analysis

To assess MUP profile diversity, urine was collected from weaning (P21) to P45 in male mice following either spontaneous urination when handled, or provoked after exerting a gentle pressure on the mouse bladder. The urine was collected in microcentrifuge tubes kept on ice during the collection procedure. All samples were initially frozen at -20°C then kept at -80°C until further processing. For protein analysis, 1 µl of urine was mixed with 1X sample buffer (Invitrogen) and 1X reducing agent (Invitrogen). Samples were boiled for 5 min and electrophoresed for 75 min at 150 V in 4-12% MES SDS-polyacrylamide gels according to the protocol supplied with the NuPAGE system (Invitrogen). After the migration, the proteins were transferred onto 0.2 µm nitrocellulose membrane (Invitrogen) in the blot module of the NuPAGE system (Invitrogen) for 90 min at 30V in cold conditions. Membranes were then blocked for 1 hour in blocking buffer [(TBS with 0.05% Tween 20 (TBST) and 5% non-fat dry milk] at RT, and incubated for 48 hour at 4°C with the primary antibody diluted in blocking buffer (rabbit polyclonal anti-MUP1, 1:200 dilution, sc-66976, Santa Cruz Biotechnology, INC). Following this, membranes were washed three times with 1X TBST before incubation with the secondary antibody (peroxidase anti-Rabbit IgG (H+L), 1:2000 dilution, PI-1000, Vector Laboratories) diluted in blocking buffer for 1 hour at RT. After incubation with secondary antibody, the membranes were washed three times with 1X TBST. Immunoreactions were developed using the ECL detection kit (NEL101; PerkinElmer, Boston, MA) and scanned using a desktop scanner (Epson Expression 1680 PRO).

### Tissue protein extraction and western blot analyses

Both hippocampus and cortex from adult Ts65dn and WT mice were sonicated in 400 µL (for hippocampus) or 800 µL (for cortex) of lysis buffer (10 mM Tris pH 7.4, 10% sucrose and proteases inhibitors (1 pellet for 10mL Complete; Roche Diagnostics GmbH)) and stored at -80°C until use. Protein concentration was determined using the BCA assay (Pierce), subsequently diluted with 2X LDS (Life) and supplemented with reducing agent (Life). Samples were boiled for 10min at 100°C. Proteins were separated onto precast 12% Criterion XT Bis-Tris polyacrylamide gels (Bio-Rad) using 1X MOPS SDS running buffer. Subsequently, proteins were transferred onto a 0.4 µm nitrocellulose membrane (G&E Healthcare). For low molecular weight proteins, such as carboxy-terminal fragments of APP (CTFs), 16,5% Criterion XT Tris-Tricine polyacrylamide gels (Bio- Rad) in 1X Tris-Tricine SDS running buffer were used. These were transferred onto a 0.2 µm nitrocellulose membrane (G&E Healthcare). For estimation of molecular weights, a molecular weight marker (Novex and Magic Marks, Life Technologies) was used. Membranes were incubated in blocking buffer [TNT (Tris 15 mM pH 8, NaCl 140 mM, 0.05% Tween) and 5% non-fat dry milk or 5% BSA] at RT and incubated overnight at 4°C with the appropriate primary antibody (supplementary table S2) diluted in blocking buffer (TNT with 5% Milk or BSA). Following this, membranes were incubated with corresponding secondary antibodies (supplementary table S2). Immunoreactions were developed using using chemiluminescence kits (ECLTM, Amersham Bioscience) and visualized using a LAS3000 imaging system (Fujifilm). Results were normalized to GAPDH and quantification was performed using ImageJ software (Scion Software).

### Orchidectomy

Adult males were subjected to bilateral gonadectomy via the scrotal route, under isofluorane anesthesia.

### Behavioral studies

**Habituation/dishabituation test.** The habituation/dishabituation test was used to assess the ability to differentiate between different odours (Breton-Provencher et al., 2009). Mice were single-housed for 8 days prior to testing. This olfactory test included a presentation of acetophenone (00790, Sigma) for habituation and octantal (05608, Sigma) for dishabituation, or vice versa. Before the test, mice were allowed to explore the open-field area and an empty odour box for 30 min. After this habituation period, mice were sequentially presented with one odour for four consecutive trials for a duration of 1 min, and an inter-trial interval of 10 min was maintained to ensure the replacement of the odour. After four consecutive trials, a second odour was presented during a 1 min trial. Odours (20µl of 1:1000 dilution) were administered on a filter paper and placed in a perforated plastic box to avoid direct contact with the odour stimulus. The measurement consisted of recording the total amount of time the mouse spent sniffing the object during different trials.

**Novel object recognition test.** Recognition memory was assessed using the novel object recognition test (Leger et al., 2013). Mice were single-housed for 5 days prior to testing. On day 1, two identical objects (A+A) were placed within the open-field arena on opposite sides of the cage, equidistant from the cage walls. Each mouse was placed within the two objects and allowed to explore them for 15 min. Day 2 consisted of two phases, a familiarization and a test phase. During the familiarization phase (trial 1) that lasted 15 min, mice explored two other identical objects (B+B). After this phase, mice were placed back in its home cage for 1 hour before starting the test phase. During the test phase, one object from trial 1 and a completely new object (B+C) were placed within the open-field area and mice were allowed to explore them for 5 min (trial 2). The object recognition score was calculated as the time spent exploring the new object (trial 2) over the total exploration time, and is used to represent recognition memory function.

**Y-maze test.** Natural spontaneous exploratory behavior and visuospatial short-term memory were tested using the Y-maze (Bridoux et al., 2013; Dellu et al., 2000). The Y-maze consisted of three white wooden arms (24.0 cm x 6.5 cm x 15 cm), elevated to a height of 41.0 cm above the floor and was surrounded with visual cues on the wall. Mice were placed in the start arm, facing the end of this arm, and were allowed to explore the maze for 10 min while one arm was blocked (novel arm). Consequently, mice were placed in their home cage for 1 h before being allowed to explore all three arms for 5 min. Trajectories of the mice were recorded using EthoVision video tracking equipment and software (Noldus Bv, Wageningen, The Netherlands). The time spent in the novel arm and latency to enter the novel arm were compared between mice.

### Brain tissue dissection

Mice were euthanized by decapitation and trunk blood was collected for hormone level analyses. The preoptic area (POA) of the hypothalamus was dissected using Wecker scissors (Moria, France) under a binocular magnifying glass, placed in dry ice immediately and stored at - 80°C until further processing and assays.

### RNA isolation from POA and quantitative RT-PCR analyses

Total RNA, containing mRNA and miRNA, was extracted with the Ambion mirVana^{™} miRNA Isolation Kit (Ambion, Inc; CA, USA) by trituration of the fragments through 22 and 26 gauge needles in succession. Quality and concentration of RNAs were determined by spectrophotometer ND-1000 NANODROP 385 (Thermo-scientific). For gene expression analyses, mRNAs were reverse transcribed using SuperScript^{®} III Reverse Transcriptase (Life Technologies). Real-time PCR was carried out on Applied Biosystems 7900HT Fast Real-Time PCR System using exon-boundary-specific TaqMan^{®} Gene Expression Assays (Applied Biosystems) (supplementary table S3).

MicroRNA expression analyses were performed using TaqMan specific RT primers and the TaqMan miRNA Reverse Transcription Kit (Applied Biosystems). Thereafter, quantitative real-time PCRs were performed using predesigned assays for miRNAs (Applied Biosystems) (supplementary table S3) on an Applied Biosystems 7900HT thermocycler using the manufacturer's recommended cycling conditions. Gene and miRNA expression data were analyzed using SDS 2.4.1 and Data Assist 3.0.1 software (Applied Biosystems).

### Isolation of hypothalamic GnRH neurons using Fluorescence-Activated Cell Sorting and Quantitative RT-PCR analyses

The preoptic regions of Gnrh::Gfp and Gnrh::Gfp;Ts65dn mice were microdissected and enzymatically dissociated using a Papain Dissociation System (Worthington, Lakewood, NJ) to obtain single-cell suspensions. FACS was performed using an EPICS ALTRA Cell Sorter Cytometer device (BD Bioscience). The sort decision was based on measurements of GFP fluorescence (excitation: 488nm, 50 mW; detection: GFP bandpass 530/30 nm, autofluorescence bandpass 695/40nm) by comparing cell suspensions from Gnrh::Gfp and Gnrh::Gfp;Ts65dn animals, as indicated in supplementary figure S5. For each animal, GFP positive and negative cells were sorted directly into 10 µl extraction buffer [0.1% Triton^{®} X-100 (Sigma-Aldrich) and 0.4 U/µl RNaseOUT^{™} (Life Technologies)].

In order to analyze gene expression, mRNAs obtained from FACS-sorted GnRH neurons were reverse transcribed using SuperScript^{®} III Reverse Transcriptase (Life Technologies) and a linear pre-amplification step was performed using the TaqMan^{®} PreAmp Master Mix Kit protocol (P/N 4366128, Applied Biosystems). Real-time PCR was carried out on Applied Biosystems 7900HT Fast Real-Time PCR System as described previously using specific TaqMan^{®} Gene Expression Assays (Applied Biosystems) (supplementary table S3).

MicroRNA expression analyses of FACS-sorted GnRH neurons were performed using stem-loop RT-PCR based TaqMan Rodent MicroRNA Arrays (Applied Biosystems). Briefly, miRNAs were reverse transcribed using the TaqMan miRNA Reverse Transcription Kit (Applied Biosystems) in combination with the stem-loop Megaplex primer pool A according to the manufacturer's instructions. A linear pre-amplification step was performed using the TaqMan^{®} PreAmp Master Mix Kit protocol (P/N 4366128, Applied Biosystems) and quantitative real-time PCRs were performed using TaqMan Low-Density Arrays (Applied Biosystems) on an Applied Biosystems 7900HT thermocycler using the manufacturer's recommended cycling conditions. Gene and miRNA expression data were analyzed using SDS 2.4.1 and Data Assist 3.0.1 software (Applied Biosystems).

### Preparation of donor tissues and neuronal grafting

Tissue donors for the POA grafts were obtained from postnatal day 2 (P2) WT mice, that contained GnRH neurons which release GnRH (WT-POA), and Gnrh::cre; BoNTBloxP-STOP-loxP mice, that contain GnRH neurons which do not release GnRH (BoNTBGnrh-POA).

The tissues were microdissected and enzymatically dissociated using a Papain Dissociation System (Worthington, Lakewood, NJ) to obtain a cell suspension in 5 µl 1X HBSS solution. Two preoptic tissues were used by implant.

Adult Ts65dn mice were placed in a stereotaxic frame (Kopf^{®} Instruments, California) under anesthesia (isoflurane), and a burr hole was drilled -1.7 mm from Bregma at the midline, according to a mouse brain atlas (Paxinos and Franklin, 2004). A 25 µl Hamilton syringe (22-gauge needle) was slowly inserted into the 3v (5.6 mm deep relative to the dura), and 5 µl of the different solutions which contain the WT-POA or BoNTBGnrh-POA explants were injected using an infusion pump (KD Scientific, Holliston, MA) over 10 min.

Under the same conditions, adult 65dn and WT mice were injected with 5µl of vehicle (HBSS 1X) (sham groups).

### Adeno-associated viral vectors and stereotactic infusions

For the selective overexpression of miR-200 family, particularly the member miR-200b, in the hypothalamus of adult Ts65dn males, bilateral injections (150 nl or 300 nl total) of scAAV9-EF1a-mmu-miR-200b-eGFP (AAV-miR200b, 2.1 × 1013 GC/ml) or scAAV9-EF1a-ctrl-miR-eGFP (AAV-GFP, 2.2 × 1013 GC/ml) were administrated into the POA (AP:+0.5 mm, ML:±0.12 mm, DV: -5.3 mm) at a rate of 20 nl/min through a 5 µl Hamilton syringe. The needle was left undisturbed for 5 min after the injection. Both viruses were obtained from the vector biolabs and injection coordinates were based on the Paxinos mouse brain atlas (Paxinos and Franklin, 2004).

### Brain preparation for immunohistochemical analysis

Neonatal (P0) mice, anesthetized on ice, and infantile (P12), prepubertal (P35) and adult mice, anesthetized with 50-100 mg/kg of Ketamine-HCl and 5-10mg/kg Xylazine-HCl, were perfused transcardially with 2-10 ml of saline, followed by 10-100 ml of 4% paraformaldehyde (PFA), pH7.4. Brains were collected and fixed with the same fixative for 2h at 4°C, embedded in OCT embedding medium (Tissue-Tek), frozen on dry ice, and stored at -80°C until cryosectioning.

### Immunohistochemistry and quantification

Tissues were cryosectioned (Leica cryostat) at 16 µm for P0 and at 35 µm (free-floating sections) for P12, P35 and adult brains, unless otherwise indicated.

### Assessment of GnRHprotein expression

Immunohistofluorescence experiments were carried out as previously reported (Hanchate et al., 2012; Messina et al., 2011). Coronal sections were then washed in 0.1M PBS, and incubated in blocking solution (2% goat serum + 0.5% Triton X-100) in PBS 0.1M for 60 min. Subsequently, sections were incubated in guinea pig anti-GnRH (1:10000) raised by Dr. Erik Hrabovszky, (Laboratory of Endocrine Neurobiology, Institute of Experimental Medicine of the Hungarian Academy of Sciences, Budapest, Hungary) (Hrabovszky et al., 2011) in the case of P0 brains; and in rabbit anti-GnRH (1:3000), a generous gift from Prof. G. Tramu (Centre Nationale de la Recherche Scientifique, URA 339, Université Bordeaux I, Talence, France) (Beauvillain and Tramu, 1980), in the case of P12, P35 and adult brains, in blocking solution for 48 hour at 4°C. After incubation in the primary antibody, sections were rinsed with 0.1M PBS three times for 10 min each and then incubated with Alexa fluor 568 conjugated anti-guinea pig (1:500) or anti-rabbit (1/500; Invitrogen A11077) secondary antibodies for 90 min at RT. Sections were then washed, counterstained with Hoechst (1:10,000; Thermo Fisher Scientific Cat# H3569, RRID:AB_2651133) for 3 min, rinsed with 0.1M PBS three times for 10 min and mounted with coverslips using Mowiol coverslip mounting solution. Because the GnRH neuronal population is very limited in the mouse brain, all neurons were counted by eyes under the microscope in one out of two series of brain (P12, P35 and adult) or head (P0) sections. Images were acquired using a Zeiss Axio Imager Z2 ApoTome microscope (Zeiss, Germany).

### Analysis of vGaT or vGluT2 appositions on GnRH neurons

Coronal sections from P12 and P35 mice were washed in 0.1M PBS three times for 10 min, and incubated with blocking solution [0.1M PBS, 0.25% bovine serum albumin (BSA; Sigma, A9418), 0.3% Triton X-100 (Sigma, T8787) with 10% normal donkey serum (NDS; Sigma, D9663)] for 90 min at RT. Sections were then incubated in rabbit anti-GnRH (1:6,000), a generous gift from Prof. G. Tramu (Centre Nationale de la Recherche Scientifique, URA339, Université Bordeaux I, Talence, France) ) (Beauvillain and Tramu, 1980) and guinea pig anti-vGaT (1:750, Synaptic Systems, 131 004) or vGluT2 (1:750, Synaptic Systems, 135 404) in blocking solution for 72 hours at 4°C. After the incubation with the primary antibodies, sections were rinsed with 0.1M PBS three times for 10 min and incubated with the corresponding secondary antibodies, i.e., Alexa fluor 488 conjugated donkey anti-rabbit (1:400; Life Technologies, Molecular Probes, Invitrogen, A21206) and Alexa fluor 594 conjugated donkey anti-guinea pig antibody (1:400; Jackson Immunoresearch, 706-585-148) for 90 min in 0.1M PBS at RT. Subsequently, sections were washed in 0.1M PBS three times for 10 min and incubated with Hoechst (1:10,000; Thermo Fisher Scientific, H3569, RRID:AB_2651133) for 3 min, followed by washing with 0.1M PBS three times for 10 min. Lastly, the sections were mounted with coverslips using Mowiol coverslip mounting solution. Images were acquired using a LSM 710 Zeiss upright confocal laser-scanning microscope equipped with LSM 710 software (Zeiss, Germany).

### Assessment of Adeno-associated viral vector

Coronal sections were then washed with 0.1M PBS, and incubated with blocking solution [(5% donkey serum + 0.5% Triton X-100) in 0.1M PBS] for 60 min. The sections were then incubated with chicken anti-GFP (1:500; Aves Labs, Inc GFP-1020) and rabbit anti-GnRH (1:3000), a generous gift from Prof. G. Tramu (Centre Nationale de la Recherche Scientifique, URA 339, Université Bordeaux I, Talence, France) (Beauvillain and Tramu, 1980), in blocking solution for 48 hours at 4°C. Following this, sections were rinsed with 0.1M PBS three times for 10 min each and then incubated with the secondary antibody Alexa fluor 488 conjugated donkey anti-chicken (1:500; Jackson Immuno Research 703-545-155) and Alexa 568 conjugated donkey anti-rabbit (1:500; Invitrogen A10042) for 90 min at RT. Sections were then washed, counterstained with Hoechst (1:10,000; Thermo Fisher Scientific Cat# H3569, RRID:AB_2651133) for 3 min, rinsed with 0.1M PBS three times for 10 min and mounted with coverslips using Mowiol coverslip mounting solution. Images were acquired using a LSM 710 Zeiss upright confocal laser-scanning microscope equipped with LSM 710 software (Zeiss, Germany).

### iDisco

iDisco is a solvent-based clearing method that renders brain tissue transparent while preserving fluorescence (Erturk et al., 2012; Erturk and Bradke, 2013).

Sample pre-treatment with methanol: Samples were washed in PBS (twice for 1 hour), followed by incubation in 50% methanol in 0.1M PBS (once for 1 hour), 80% methanol (once for 1 hour) and 100% methanol (twice for 1 hour). Next, samples were bleached in 5% H2O2 in 20% DMSO/methanol (2ml 30% H2O2/2ml DMSO/8ml methanol, ice cold) at 4°C overnight. Following this, samples were washed in methanol (twice for 1 hour), in 20% DMSO/methanol (twice for 1 hour), 80% methanol (once for 1 hour), 50% methanol (once for 1 hour), PBS (twice for 1 hour), and finally, PBS/0.2% TritonX-100 (twice for 1 hour) before proceeding to the staining procedures.

Whole-mount immunostaining: Samples were incubated at 37°C on an adjustable rotator in 10 ml of blocking solution (PBSGNaT) [1X PBS containing 0.2% gelatin (Sigma), 0.5% Triton X-100 (Sigma-Aldrich) and 0.01% NaAzide (Casoni et al., 2016)] for 3 nights. Samples were transferred to 10 ml of PBSGNaT containing primary antibodies (Table S1) and placed at 37°C in rotation for 7 days. This was followed by six washes of 30 min in PBSGT at RT and a final wash in PBSGT overnight at 4°C. Next, samples were incubated with secondary antibodies (1:400, Alexa 568, Alexa 647) diluted in 10 ml PBSGNaT for 2 days at 37°C in a rotating tube. After six 30 min washes in 0.1M PBS at RT, the samples were stored in PBS at 4°C in the dark until clearing.

Tissue clearing: All incubation steps were performed at RT in a fume hood, on a tube rotator at 14 rpm covered with aluminum foil to avoid contact with light. Samples were dehydrated in a graded series (20%, 40%, 60%, 80% and 100%) of Methanol (Sigma-Aldrich) diluted in H2O for 1 hour. This was followed by a delipidation step of 30-40 min in 100 % dichloromethane (DCM; Sigma-Aldrich). Samples were cleared in dibenzylether (DBE; Sigma-Aldrich) for 2h at RT on constant agitation and in the dark. Finally, samples were moved into fresh DBE and stored in glass tube in the dark at RT until imaging. We were able to image samples, as described below, without any significant fluorescence loss for up to 6 months.

### Digital image acquisition

The different immunohistofluorescence experiments described before were analyzed using one of the microscopes mentioned below and Adobe Photoshop (Adobe Systems, San Jose, CA, RRID:SCR_014199) was used to process the images.

### Fluorescence microscopy

Unless otherwise indicated, sections were analysed using a Zeiss Axio Imager Z2 ApoTome microscope (Zeiss, Germany, equipped with a motorized stage and an AxioCam MRm camera (Zeiss, Germany). Specific beam splitter (BS), excitation (Ex) and emission (Em) wavelengths were used for the visualization of green (Alexa 488-BS: 495 nm, Ex: 450/490 nm, Em: 500/550 nm), red (Alexa 688-BS: 570 nm, Ex: 538/562 nm, Em: 570/640 nm), far red (Alexa 647-BS: 660 nm, Ex: 625/655 nm, Em: 665/715 nm) and nuclear staining (Hoechst-BS: 395 nm, Ex: 335/383 nm, Em: 420/470 nm).To create photomontages, single-plane images were captured sequentially for each fluorophore using the MosaiX module of the AxioVision 4.6 system (Zeiss, Germany) and a Zeiss 20x objective (numerical aperture NA=0.80). High magnification photomicrographs represent maximal intensity projections derived from a series of triple- ApoTome adjacent images collected using the Z-stack module of the AxioVision 4.6 system. All images were captured in a stepwise fashion over a defined z-focus range corresponding to all visible staining within the section and consistent with the optimum step size for the corresponding objective and the wavelength.

### Confocal imaging

Sections from the analysis of vGaT and vGluT2 appositions on GnRH neurons were imaged using a LSM 710 Zeiss upright confocal laser-scanning microscope equipped with LSM 710 software. For each GnRH-IR cell, a stack of images at 0.25 µm intervals were collected using a 100x objective and 2x digital zoom throughout the entire depth of the GnRH-IR neuron. A Z-series stack of images using a 100x objective were generated to estimate the density of vGaT or vGluT2 appositions. A contact was defined when there were no black pixels between the primary GnRH-IR dendritic spine and the vGaT-positive or vGluT2-positive terminal. For each image, the number of vGaT- or vGluT2-labeled puncta directly opposed to GnRH-IR neuronal soma and dendrite (up to 45 µm along the GnRH primary dendrite) were counted and combined to provide the mean values for each cell. The primary GnRH-IR dendrite could not be followed for more than 45 µm from the cell body, and therefore we determined the number of vGaT appositions every 15 µm until the dendrite exited the section. Data are presented as vGaT or vGluT2 appositions/µm.

### Light sheet imaging

3D imaging was performed as previously described (Belle et al., 2014). An ultramicroscope (LaVision BioTec) using ImspectorPro software (LaVision BioTec) was used to perform imaging. The light sheet was generated by a laser (wavelength 488 or 561 nm, Coherent Sapphire Laser, LaVision BioTec) and two cylindrical lenses. A binocular stereomicroscope (MXV10, Olympus) with a 2x objective (MVPLAPO, Olympus) was used at different magnifications (1.6x, 4x, 5x, and 6.3x). Samples were placed in an imaging reservoir made of 100% quartz (LaVision BioTec) filled with DBE and illuminated from the side by the laser light. A PCO Edge SCMOS CCD camera (2,560 × 2,160 pixel size, LaVision BioTec) was used to acquire images. The step size between each image was fixed at 2 µm.

### Acute GnRH injections

In order to study the effect of GnRH on cognitive and olfactory performance, adult male Ts65Dn mice grafted with a POA explant from iBot mice (65dn+POA-TOX) were treated with GnRH-1 peptide (Genecust), in a dose of 0,05 µg/g of BW, or vehicle (PBS pH 7,4).

To test the olfactory discrimination capacity, the animals received the one single intraperitoneal (ip) injection of GnRH-1 or vehicle 2 hours before the habituation phase. In the case of NOR test, on day 1, the animals received two ip injections of GnRH-1 peptide or vehicle. The first injection was given 2 hours before the start of the trial, and a second one 12 hours after the first injection to promote memory consolidation. On day 2, the animals received the ip injection 2 hours before the start of the first trial.

### Continuous and pulsatile subcutaneous infusion

Adult mice were implanted with osmotic minipumps (1002, Alzet, USA) receiving continuous infusion of vehicle (sterile 0.1M PBS) or LUTRELEF^{®} (0.25 µgr/ 3h) (Ferring Pharmaceuticals, Switzerland); or with a programmable micro infusion pump (SMP-300, iPRECIO, Japan) receiving pulsatile infusion of vehicle or LUTRELEF^{®} (every 3 hours, a peak of 0.25 µg during 10 min), mimicking GnRH/LH pulsatility reported in WT mice (Czieselsky et al., 2016), and a basal infusion with a low dose (0.0025 µg/10 min) for the rest of the time,. The pumps were placed under the skin on the back of the mouse. Both olfactory and cognitive deficiencies were confirmed before in these animals. One week after the surgery, mice were retested to evaluate their olfactory and cognitive performance. Two weeks after the surgery, repetitive tail-tip blood sampling was undertaken (as described elsewhere) to assess the LH pulsatility profile.

### Sample size and randomization statement

Sample sizes for physiological and neuroanatomical studies and for miRNA and gene expression analyses were estimated based on past experience and those presented in the literature. Mice from at least three different litters from each group were used to study sexual maturation, fertility and used to perform quantitative RT-PCR analyses in cells isolated by FACS, anatomy and immunostaining. No randomization method was used to assign subjects in the experimental groups or to collect and process data.

### Presentation of data and statistics

All statistical analyses were performed using Prism 7 (GraphPad Software) and assessed for normality (Shapiro-Wilk test) and variance, at appropriate places. Sample sizes were chosen according to standard practice in the field. Data were compared using an unpaired/paired two-tailed Student's t test, a Mann-Whitney U test, or a one-way ANOVA for multiple comparisons. A Tukey's post hoc test was performed when appropriate. The significance level was set at p < 0.05. Data are indicated as means ± s.e.m. The number of biologically independent experiments, P values and degrees of freedom are indicated either in the main text or in the figure legends.

### Example 2

### Acute chemogenetic inhibition of GnRH-R expressing neurons impairs cognitive and olfactory performance in adult control mice.

Neuroanatomical results demonstrated that extrahypothalamic GnRH projections are found in brain areas controlling cognitive and social behaviors. Furthermore, the potential role of GnRH in the regulation of non-reproductive processes is also consistent with the three-dimensional (3D) imaging of solvent-cleared (iDISCO) analyses performed by the inventors identifying GFP-labeled neurons expressing the GnRH Receptor (GnRH-R) in the cortex and hippocampus of the murine brain.

Intriguingly, as shown in **Figure 5****,** when neurons expressing the GnRH receptor, GnRH-R, in the hippocampus of *Gnrhr::Cre* mice were infected with an inhibitory DREADD viral vector (AAV8-hSYN-DIO-hM4D(Gi)-mCherry) **(****Figure 5 A****),** a single injection of 200 µl of clozapine-n-oxide (CNO - 3 mg/kg) dramatically reduced both cognitive and olfactory performance in wild type (wt) mice **(****Figure 5** **B and C).** Taken together, these data further highlight the intriguing idea that normal cognitive and olfactory function depend on GnRH action in target regions far removed from the hypothalamus, and that the acquired deficiency of extrahypothalamic GnRH fibers in Ts65Dn mice during postnatal development may play a crucial role in their DS-like phenotype.

### References

Hayes, F., Dwyer, A., & Pitteloud, N. (2013). Hypogonadotropic hypogonadism (HH) and gonadotropin therapy. In Endotext [Internet]. MDText. com, Inc.
Leyendecker G, Wildt L, Hansmann M (1980) Pregnancies following chronic intermittent (pulsatile) administration of GnRH by means of a portable pump (Zyklomat) - a new approach to the treatment of infertility in hypothalamic amenorrhea. J Clin Endocrinol Metab 51: 1214-1216
Shoemaker J, Simons AHM, van Osnabrugge GJC, Lugtenburg C, van Kessel H (1981) Pregnancy after prolonged pulsatile administration of luteinizing hormone-releasing hormone in a patient with clomiphen-resistant secondary amenorrhea. J Clin Endocrinol Metab 52: 882-885
Reid RL, Leopold GR, Yen SSC (1981) Induction of ovulation and pregnancy with pulsatile luteinizing hormone releasing factor: Dosage and mode of delivery. Fertil Steril 36: 553-559.
Boehm, Ulrich, et al. "Expert consensus document: European Consensus Statement on congenital hypogonadotropic hypogonadism-pathogenesis, diagnosis and treatment." Nature Reviews Endocrinology 11.9 (2015): 547.
Sachdev, Perminder S., et al. "Classifying neurocognitive disorders: the DSM-5 approach." Nature Reviews Neurology 10.11 (2014): 634.
Wilson, Robert S., et al. "Olfactory identification and incidence of mild cognitive impairment in older age." Archives of general psychiatry 64.7 (2007): 802-808.
Sperling, Reisa A., et al. "Toward defining the preclinical stages of Alzheimer's disease: Recommendations from the National Institute on Aging-Alzheimer's Association workgroups on diagnostic guidelines for Alzheimer's disease." Alzheimer's & dementia 7.3 (2011): 280-292.
Ross, G. Webster, et al. "Association of olfactory dysfunction with risk for future Parkinson's disease." Annals of neurology 63.2 (2008): 167-173.
Doty, R. L. "Office procedures for quantitative assessment of olfactory function". Am. J. Rhinol. 21, 460-473 (2007).
Eibenstein, A. et al. « Modern psychophysical tests to assess olfactory function". Neurol. Sci. 26, 147-155 (2005).
Kobal, G. & Hummel, T. "Olfactory (chemosensory) event-related potentials". Toxicol. Ind. Health 10, 587-596 (1994).
Lund, Carina, et al. "Development of gonadotropin-releasing hormone-secreting neurons from human pluripotent stem cells." Stem cell reports 7.2 (2016): 149-157.
Ahmed, M.M., Sturgeon, X., Ellison, M., Davisson, M.T., and Gardiner, K.J. (2012). Loss of correlations among proteins in brains of the Ts65Dn mouse model of down syndrome. J Proteome Res 11, 1251-1263.
Antonarakis, S.E. (2017). Down syndrome and the complexity of genome dosage imbalance. Nat Rev Genet 18, 147-163.
Ballard, C., Mobley, W., Hardy, J., Williams, G., and Corbett, A. (2016). Dementia in Down's syndrome. Lancet Neurol 15, 622-636.
Bayen, E., Possin, K.L., Chen, Y., Cleret de Langavant, L., and Yaffe, K. (2018). Prevalence of Aging, Dementia, and Multimorbidity in Older Adults With Down Syndrome. JAMA Neurol 75, 1399-1406.
Beauvillain, J.C., and Tramu, G. (1980). Immunocytochemical demonstration of LH-RH, somatostatin, and ACTH-like peptide in osmium-postfixed, resin-embedded median eminence. J.Histochem.Cytochem. 28, 1014-1017.
Belchetz, P.E., Plant, T.M., Nakai, Y., Keogh, E.J., and Knobil, E. (1978). Hypophysial responses to continuous and intermittent delivery of hypopthalamic gonadotropin-releasing hormone. Science 202, 631-633.
Belle, M., Godefroy, D., Couly, G., Malone, S.A., Collier, F., Giacobini, P., and Chedotal, A. (2017). Tridimensional Visualization and Analysis of Early Human Development. Cell 169, 161-173 e112.
Belle, M., Godefroy, D., Dominici, C., Heitz-Marchaland, C., Zelina, P., Hellal, F., Bradke, F., and Chedotal, A. (2014). A simple method for 3D analysis of immunolabeled axonal tracts in a transparent nervous system. Cell Rep 9, 1191-1201.
Bianchi, P., Bettini, S., Guidi, S., Ciani, E., Trazzi, S., Stagni, F., Ragazzi, E., Franceschini, V., and Bartesaghi, R. (2014). Age-related impairment of olfactory bulb neurogenesis in the Ts65Dn mouse model of Down syndrome. Exp Neurol 251, 1-11.
Boehm, U., Bouloux, P.M., Dattani, M.T., de Roux, N., Dode, C., Dunkel, L., Dwyer, A.A., Giacobini, P., Hardelin, J.P., Juul, A., et al. (2015). Expert consensus document: European Consensus Statement on congenital hypogonadotropic hypogonadism--pathogenesis, diagnosis and treatment. Nat Rev Endocrinol 11, 547-564.
Breton-Provencher, V., Lemasson, M., Peralta, M.R., 3rd, and Saghatelyan, A. (2009). Interneurons produced in adulthood are required for the normal functioning of the olfactory bulb network and for the execution of selected olfactory behaviors. J Neurosci 29, 15245-15257.
Bridoux, A., Laloux, C., Derambure, P., Bordet, R., and Monaca Charley, C. (2013). The acute inhibition of rapid eye movement sleep by citalopram may impair spatial learning and passive avoidance in mice. J Neural Transm (Vienna) 120, 383-389.
Casoni, F., Malone, S.A, Belle, M., Luzzati, F., Collier, F., Allet, C., Hrabovszky, E., Rasika, S., Prevot, V., Chedotal, A., et al. (2016). Development of the neurons controlling fertility in humans: new insights from 3D imaging and transparent fetal brains. Development 143, 3969-3981.
Charlton, H.M., Jones, A.J., Whitworth, D., Gibson, M.J., Kokoris, G., Zimmerman, E.A., and Silverman, A.J. (1987). The effects of the age of intracerebroventricular grafts of normal preoptic area tissue upon pituitary and gonadal function in hypogonadal (HPG) mice. Neuroscience 21, 175-181.
Choi, J.H., Berger, J.D., Mazzella, M.J., Morales-Corraliza, J., Cataldo, A.M., Nixon, R.A., Ginsberg, S.D., Levy, E., and Mathews, P.M. (2009). Age-dependent dysregulation of brain amyloid precursor protein in the Ts65Dn Down syndrome mouse model. J Neurochem 110, 1818-1827.
Clarke, I.J., and Cummins, J.T. (1982). The temporal relationship between gonadotropin releasing hormone (GnRH) and luteinizing hormone (LH) secretion in ovariectomized ewes. Endocrinology 111, 1737-1739.
Czieselsky, K., Prescott, M., Porteous, R., Campos, P., Clarkson, J., Steyn, F.J., Campbell, R.E., and Herbison, A.E. (2016). Pulse and Surge Profiles of Luteinizing Hormone Secretion in the Mouse. Endocrinology 157, 4794-4802.
Dellu, F., Contarino, A., Simon, H., Koob, G.F., and Gold, L.H. (2000). Genetic differences in response to novelty and spatial memory using a two-trial recognition task in mice. Neurobiol Learn Mem 73, 31-48.
Deslypere, J.P., Kaufman, J.M., Vermeulen, T., Vogelaers, D., Vandalem, J.L., and Vermeulen, A. (1987). Influence of age on pulsatile luteinizing hormone release and responsiveness of the gonadotrophs to sex hormone feedback in men. J Clin Endocrinol Metab 64, 68-73.
Doty, R.L. (2012). Olfactory dysfunction in Parkinson disease. Nat Rev Neurol 8, 329-339.
Editorial, T.L. (2013). Strengthening connections between Down syndrome and AD. 12, 931.
Elton, T.S., Sansom, S.E., and Martin, M.M. (2010). Trisomy-21 gene dosage over-expression of miRNAs results in the haploinsufficiency of specific target proteins. RNA Biol 7, 540-547.
Epstein, C.J., Korenberg, J.R., Anneren, G., Antonarakis, S.E., Ayme, S., Courchesne, E., Epstein, L.B., Fowler, A., Groner, Y., Huret, J.L., et al. (1991). Protocols to establish genotype-phenotype correlations in Down syndrome. Am J Hum Genet 49, 207-235.
Erturk, A., Becker, K., Jahrling, N., Mauch, C.P., Hojer, C.D., Egen, J.G., Hellal, F., Bradke, F., Sheng, M., and Dodt, H.U. (2012). Three-dimensional imaging of solvent-cleared organs using 3DISCO. Nat Protoc 7, 1983-1995.
Erturk, A., and Bradke, F. (2013). High-resolution imaging of entire organs by 3-dimensional imaging of solvent cleared organs (3DISCO). Exp Neurol 242, 57-64.
Garcia-Galiano, D., van Ingen Schenau, D., Leon, S., Krajnc-Franken, M.A., Manfredi-Lozano, M., Romero-Ruiz, A., Navarro, V.M., Gaytan, F., van Noort, P.I., Pinilla, L., et al. (2012). Kisspeptin signaling is indispensable for neurokinin B, but not glutamate, stimulation of gonadotropin secretion in mice. Endocrinology 153, 316-328.
Granger, A., Ngo-Muller, V., Bleux, C., Guigon, C., Pincas, H., Magre, S., Daegelen, D., Tixier-Vidal, A., Counis, R., and Laverriere, J.N. (2004). The promoter of the rat gonadotropin-releasing hormone receptor gene directs the expression of the human placental alkaline phosphatase reporter gene in gonadotrope cells in the anterior pituitary gland as well as in multiple extrapituitary tissues. Endocrinology 145, 983-993.
Hall, J.E., Lavoie, H.B., Marsh, E.E., and Martin, K.A. (2000). Decrease in gonadotropin-releasing hormone (GnRH) pulse frequency with aging in postmenopausal women. J Clin Endocrinol Metab 85, 1794-1800.
Hanchate, N.K., Giacobini, P., Lhuillier, P., Parkash, J., Espy, C., Fouveaut, C., Leroy, C., Baron, S., Campagne, C., Vanacker, C., et al. (2012). SEMA3A, a Gene Involved in Axonal Pathfinding, Is Mutated in Patients with Kallmann Syndrome. PLoS Genet 8, e1002896.
Harfe, B.D., McManus, M.T., Mansfield, J.H., Hornstein, E., and Tabin, C.J. (2005). The RNaseIII enzyme Dicer is required for morphogenesis but not patterning of the vertebrate limb. Proc Natl Acad Sci U S A 102, 10898-10903.
Hrabovszky, E., Molnar, C.S., Sipos, M.T., Vida, B., Ciofi, P., Borsay, B.A., Sarkadi, L., Herczeg, L., Bloom, S.R., Ghatei, M.A., et al. (2011). Sexual dimorphism of kisspeptin and neurokinin B immunoreactive neurons in the infundibular nucleus of aged men and women. Front Endocrinol (Lausanne) 2, 80.
Hsiang, Y.H., Berkovitz, G.D., Bland, G.L., Migeon, C.J., and Warren, A.C. (1987). Gonadal function in patients with Down syndrome. Am J Med Genet 27, 449-458.
Kuhn, D.E., Nuovo, G.J., Terry, A.V., Jr., Martin, M.M., Malana, G.E., Sansom, S.E., Pleister, A.P., Beck, W.D., Head, E., Feldman, D.S., et al. (2010). Chromosome 21-derived microRNAs provide an etiological basis for aberrant protein expression in human Down syndrome brains. J Biol Chem 285, 1529-1543.
Leger, M., Quiedeville, A., Bouet, V., Haelewyn, B., Boulouard, M., Schumann-Bard, P., and Freret, T. (2013). Object recognition test in mice. Nat Protoc 8, 2531-2537.
Lott, I.T., and Head, E. (2019). Dementia in Down syndrome: unique insights for Alzheimer disease research. Nat Rev Neurol 15, 135-147.
Manly, J.J., Merchant, C.A., Jacobs, D.M., Small, S.A., Bell, K., Ferin, M., and Mayeux, R. (2000). Endogenous estrogen levels and Alzheimer's disease among postmenopausal women. Neurology 54, 833-837.
Messina, A., Ferraris, N., Wray, S., Cagnoni, G., Donohue, D.E., Casoni, F., Kramer, P.R., Derijck, A.A., Adolfs, Y., Fasolo, A., et al. (2011). Dysregulation of Semaphorin7A/beta1-integrin signaling leads to defective GnRH-1 cell migration, abnormal gonadal development and altered fertility. Hum Mol Genet 20, 4759-4774.
Messina, A., Langlet, F., Chachlaki, K., Roa, J., Rasika, S., Jouy, N., Gallet, S., Gaytan, F., Parkash, J., Tena-Sempere, M., et al. (2016). A microRNA switch regulates the rise in hypothalamic GnRH production before puberty. Nat Neurosci 19, 835-844.
Moffat, S.D., Zonderman, A.B., Metter, E.J., Kawas, C., Blackman, M.R., Harman, S.M., and Resnick, S.M. (2004). Free testosterone and risk for Alzheimer disease in older men. Neurology 62, 188-193.
Moore, A.M., Prescott, M., Marshall, C.J., Yip, S.H., and Campbell, R.E. (2015). Enhancement of a robust arcuate GABAergic input to gonadotropin-releasing hormone neurons in a model of polycystic ovarian syndrome. Proc Natl Acad Sci U S A 112, 596-601.
Moore, C.S., Hawkins, C., Franca, A., Lawler, A., Devenney, B., Das, I., and Reeves, R.H. (2010). Increased male reproductive success in Ts65Dn "Down syndrome" mice. Mamm Genome 21, 543-549.
Nijjar, R.K., and Murphy, C. (2002). Olfactory impairment increases as a function of age in persons with Down syndrome. Neurobiol Aging 23, 65-73.
Prevot, V. (2015). Puberty in mice and rats. In in Knobil and Neill's Physiology of Reproduction. T.M. Plant, and J. Zeleznik, eds. (New York: Elsevier,), pp. 1395-1439
Rance, N.E., Young, W.S., 3rd, and McMullen, N.T. (1994). Topography of neurons expressing luteinizing hormone-releasing hormone gene transcripts in the human hypothalamus and basal forebrain. J Comp Neurol 339, 573-586.
Risser, J.M., and Slotnick, B.M. (1987). Nipple attachment and survival in neonatal olfactory bulbectomized rats. Physiol Behav 40, 545-549.
Reeves, R.H., Irving, N.G., Moran, T.H., Wohn, A., Kitt, C., Sisodia, S.S., Schmidt, C., Bronson, R.T., and Davisson, M.T. (1995). A mouse model for Down syndrome exhibits learning and behaviour deficits. Nat Genet 11, 177-184.
Reinholdt, L.G., Ding, Y., Gilbert, G.J., Czechanski, A., Solzak, J.P., Roper, R.J., Johnson, M.T., Donahue, L.R., Lutz, C., and Davisson, M.T. (2011). Molecular characterization of the translocation breakpoints in the Down syndrome mouse model Ts65Dn. Mamm Genome 22, 685-691.
Schapiro, M.B., Haxby, J.V., Grady, C.L., Duara, R., Schlageter, N.L., White, B., Moore, A., Sundaram, M., Larson, S.M., and Rapoport, S.I. (1987). Decline in cerebral glucose utilisation and cognitive function with aging in Down's syndrome. J Neurol Neurosurg Psychiatry 50, 766-774.
Sergeant, N., David, J.P., Champain, D., Ghestem, A., Wattez, A., and Delacourte, A. (2002). Progressive decrease of amyloid precursor protein carboxy terminal fragments (APP-CTFs), associated with tau pathology stages, in Alzheimer's disease. J Neurochem 81, 663-672.
Slezak, M., Grosche, A., Niemiec, A., Tanimoto, N., Pannicke, T., Munch, T.A., Crocker, B., Isope, P., Hartig, W., Beck, S.C., et al. (2012). Relevance of exocytotic glutamate release from retinal glia. Neuron 74, 504-516.
Spergel, D.J., Kruth, U., Hanley, D.F., Sprengel, R., and Seeburg, P.H. (1999). GABA- and glutamate-activated channels in green fluorescent protein-tagged gonadotropin-releasing hormone neurons in transgenic mice. J.Neurosci. 19, 2037-2050.
Steyn, F.J., Wan, Y., Clarkson, J., Veldhuis, J.D., Herbison, A.E., and Chen, C. (2013). Development of a methodology for and assessment of pulsatile luteinizing hormone secretion in juvenile and adult male mice. Endocrinology 154, 4939-4945.
Studd, J.W., Chakravarti, S., and Collins, W.P. (1978). Plasma hormone profiles after the menopause and bilateral oophorectomy. Postgrad Med J 54 Suppl 2, 25-30.
Tata, B., Mimouni, N.E.H., Barbotin, A.L., Malone, S.A., Loyens, A., Pigny, P., Dewailly, D., Catteau-Jonard, S., Sundstrom-Poromaa, I., Piltonen, T.T., et al. (2018). Elevated prenatal anti-Mullerian hormone reprograms the fetus and induces polycystic ovary syndrome in adulthood. Nat Med 24, 834-846.
V, B.-S., O, C., C, M.-G., R, J., M, G., B, P., and Delacourte A (1996). AD2, a phosphorylation-dependent monoclonal antibody directed against tau proteins found in Alzheimer's disease. Brain Res Mol Brain Res. Jul;39(1-2):79-88.
Van Vugt, D.A., Diefenbach, W.D., Alston, E., and Ferin, M. (1985). Gonadotropin-releasing hormone pulses in third ventricular cerebrospinal fluid of ovariectomized rhesus monkeys: correlation with luteinizing hormone pulses. Endocrinology 117, 1550-1558.
Vidal, A., Zhang, Q., Medigue, C., Fabre, S., and Clement, F. (2012). DynPeak: an algorithm for pulse detection and frequency analysis in hormonal time series. PLoS One 7, e39001.
Wilson, A.C., Salamat, M.S., Haasl, R.J., Roche, K.M., Karande, A., Meethal, S.V., Terasawa, E., Bowen, R.L., and Atwood, C. S. (2006). Human neurons express type I GnRH receptor and respond to GnRH I by increasing luteinizing hormone expression. J Endocrinol 191, 651-663.
Yaffe, K., Lui, L.Y., Zmuda, J., and Cauley, J. (2002). Sex hormones and cognitive function in older men. J Am Geriatr Soc 50, 707-712.
Yen, S.S., and Tsai, C.C. (1971). The effect of ovariectomy on gonadotropin release. J Clin Invest 50, 1149-1153.
Yoon, H., Enquist, L.W., and Dulac, C. (2005). Olfactory inputs to hypothalamic neurons controlling reproduction and fertility. Cell 123, 669-682.
Zhang, G., Li, J., Purkayastha, S., Tang, Y., Zhang, H., Yin, Y., Li, B., Liu, G., and Cai, D. (2013). Hypothalamic programming of systemic ageing involving IKK-beta, NF-kappaB and GnRH. Nature 497, 211-216.

## Claims

1. Gonadotropin-releasing hormone (GnRH) for use in the treatment of a cognitive disorder in a patient in need thereof, wherein said GnRH is administered by pulsatile administration.

2. A miRNA for use in the treatment of a cognitive disorder in a patient in need thereof, wherein said miRNA is administered so as to induce a pulsatile expression of GnRH in said patient, and wherein said miRNA is selected from the group consisting of miR-200a, miR-200b, miR-200c, miR-141, miR-429 and miR155.

3. GnRH or miRNA for use according to claim 1 or 2, wherein said patient has an olfactory dysfunction.

4. GnRH or miRNA for use according to any one of claims 1 to 3, wherein said cognitive disorder is Down syndrome.

5. GnRH or miRNA for use according to any one of claims 1 to 3, wherein said cognitive disorder is Alzheimer disease.

6. GnRH or miRNA for use according to any one of claims 1 to 3, wherein said cognitive disorder is Parkinson disease.

7. GnRH or miRNA for use according to any one of claims 1 to 3, wherein said cognitive disorder is age-associated cognitive decline.

8. GnRH or miRNA for use according to any one of claims 1 to 6, wherein said cognitive disorder is in early stage.

9. GnRH for use according to any one of claims 1 and 3 to 8, wherein said patient is a man.

10. GnRH for use according to claim 9, wherein said pulsatile administration corresponds to an administration of 25ng/kg of GnRH every 120 minutes.

11. GnRH for use according to any one of claims 1 and 3 to 8, wherein said patient is a woman.

12. GnRH for use according to claim 11, wherein said pulsatile administration corresponds to an administration of 75 ng/kg of GnRH every 90 minutes..

13. GnRH for use according to any one of claims 1 and 3 to 12, wherein said GnRH is gonadorelin.

## Patentansprüche

1. Gonadotropin-freisetzendes Hormon (GnRH) zur Verwendung bei der Behandlung einer kognitiven Störung bei einem Patienten, welcher dessen bedarf, wobei das GnRH durch pulsatile Verabreichung verabreicht wird.

2. miRNA zur Verwendung bei der Behandlung einer kognitiven Störung bei einem Patienten, welcher dessen bedarf, wobei die miRNA so verabreicht wird, dass sie eine pulsatile Expression von GnRH bei dem Patienten induziert, und wobei die miRNA ausgewählt ist aus der Gruppe bestehend aus miR-200a, miR-200b, miR-200c, miR-141, miR-429 und miR155.

3. GnRH oder miRNA zur Verwendung nach Anspruch 1 oder 2, wobei der Patient eine olfaktorische Dysfunktion aufweist.

4. GnRH oder miRNA zur Verwendung nach einem der Ansprüche 1 bis 3, wobei die kognitive Störung Down-Syndrom ist.

5. GnRH oder miRNA zur Verwendung nach einem der Ansprüche 1 bis 3, wobei die kognitive Störung Alzheimer-Krankheit ist.

6. GnRH oder miRNA zur Verwendung nach einem der Ansprüche 1 bis 3, wobei die kognitive Störung Parkinson-Krankheit ist.

7. GnRH oder miRNA zur Verwendung nach einem der Ansprüche 1 bis 3, wobei die kognitive Störung altersassoziierter kognitiver Abbau ist.

8. GnRH oder miRNA zur Verwendung nach einem der Ansprüche 1 bis 6, wobei die kognitive Störung in einem Frühstadium ist.

9. GnRH zur Verwendung nach einem der Ansprüche 1 und 3 bis 8, wobei der Patient ein Mann ist.

10. GnRH zur Verwendung nach Anspruch 9, wobei die pulsatile Verabreichung einer Verabreichung von 25 ng/kg GnRH alle 120 Minuten entspricht.

11. GnRH zur Verwendung nach einem der Ansprüche 1 und 3 bis 8, wobei der Patient eine Frau ist.

12. GnRH zur Verwendung nach Anspruch 11, wobei die pulsatile Verabreichung einer Verabreichung von 75 ng/kg GnRH alle 90 Minuten entspricht.

13. GnRH zur Verwendung nach einem der Ansprüche 1 und 3 bis 12, wobei das GnRH Gonadorelin ist.

## Revendications

1. Hormone de libération des gonadotrophines hypophysaires (GnRH) pour son utilisation dans le traitement d'un trouble cognitif chez un patient en ayant besoin, dans laquelle ladite GnRH est administrée par administration pulsatile.

2. miARN pour son utilisation dans le traitement d'un trouble cognitif chez un patient en ayant besoin, dans lequel ledit miARN est administré de façon à induire une expression pulsatile de la GnRH chez ledit patient, et dans lequel ledit miARN est sélectionné parmi le groupe constitué de miR-200a, miR-200b, miR-200c, miR-141, miR-429 et miR155.

3. GnRH ou miARN pour son utilisation selon la revendication 1 ou 2, dans lequel ledit patient présente un dysfonctionnement olfactif.

4. GnRH ou miARN pour son utilisation selon l'une quelconque des revendications 1 à 3, dans lequel ledit trouble cognitif est le syndrome de Down.

5. GnRH ou miARN pour son utilisation selon l'une quelconque des revendications 1 à 3, dans lequel ledit trouble cognitif est la maladie d'Alzheimer.

6. GnRH ou miARN pour son utilisation selon l'une quelconque des revendications 1 à 3, dans lequel ledit trouble cognitif est la maladie de Parkinson.

7. GnRH ou miARN pour son utilisation selon l'une quelconque des revendications 1 à 3, dans lequel ledit trouble cognitif est le déclin cognitif lié à l'âge.

8. GnRH ou miARN pour son utilisation selon l'une quelconque des revendications 1 à 6, dans lequel ledit trouble cognitif est à un stade précoce.

9. GnRH pour son utilisation selon l'une quelconque des revendications 1 et 3 à 8, dans laquelle ledit patient est un homme.

10. GnRH pour son utilisation selon la revendication 9, dans laquelle ladite administration pulsatile correspond à une administration de 25 ng/kg de GnRH toutes les 120 minutes.

11. GnRH pour son utilisation selon l'une quelconque des revendications 1 et 3 à 8, dans laquelle ledit patient est une femme.

12. GnRH pour son utilisation selon la revendication 11, dans laquelle ladite administration pulsatile correspond à une administration de 75 ng/kg de GnRH toutes les 90 minutes.

13. GnRH pour son utilisation selon l'une quelconque des revendications 1 et 3 à 12, dans laquelle ladite GnRH est la gonadoréline.
